# EUROPEAN PATENT APPLICATION

(11) **EP 4 745 225 A1**
(43) Date of publication of application: **20.05.2026**
(21) Application number: 24212628.2
(22) Date of filing: 13.11.2024
(51) Int. Cl.: C12N 5/00

(54) **SERUM-FREE NUTRIENT COMPOSITION FOR THE CULTIVATION OF VERTEBRATE CELLS**

(71) Applicant: The Cultivated B. GmbH, 69124 Heidelberg (DE)
(72) Inventor: Decker, Tim, 69124 Heidelberg (DE); Pacjuk, Olga, 69124 Heidelberg (DE); Kolodziej, Matheus, 69124 Heidelberg (DE); Gräser, Katharina, 69124 Heidelberg (DE); Bali, Jitin, 69124 Heidelberg (DE)
(74) Representative: Jacobi, Markus Alexander

(57) **Abstract**

The invention relates to serum-free nutrient compositions useful for the cultivation of vertebrate cells. The nutrient compositions are suitable for cultivation of cells both in suspension and adherent cultures low formation of cell aggregates and/or high cell viability.

## Description

The invention relates to serum-free nutrient compositions which is useful e.g. for the cultivation of vertebrate cells. The nutrient compositions are suitable for cultivation of cells both in suspension and adherent cultures, low formation of cell aggregates and/or high cell viability.

Today, animal cell cultures (i.e., the cultivation of cells of vertebrate origin *in vitro*), such as vertebrate cell cultures, are of high interest for numerous applications. Cultivation of vertebrate cells in vitro has already been described for numerous industrial applications, including the development and production of therapeutic antibodies, hormones, vaccines, and cell therapy. Additionally, the production of cultivated meat is an emerging market that will substantially increase the scale at which cell cultures are used globally.

In vitro cultivation of these cells is achieved by providing the cells with essential nutrients via a nutrient-rich medium, typically applied in form of an aqueous solution. For example, the widely used, commercial DMEM/F12 medium contains 21 amino acids, 10 vitamins, glucose, iron, and zinc, among other components. While such basal media provide nitrogen and carbon sources, as well as micronutrients to a diverse range of species and cell types, they need to be supplemented by additional, often cell type-specific components.

Until today, animal sera like fetal bovine serum are widely being used as a medium supplement that adds those components, including growth factors, hormones, globular proteins, additional vitamins, fatty acids and more. However, the use of animal sera, which are obtained from processed animal blood, has several downsides. Such sera bear residual risks that these can comprise e.g. pathogens, undesired hormones, remaining contents of xenobiotics and/or medicinal agents (e.g. antibiotic). Though such risk can essentially be minimized or excluded by comprehensive controls, this is still undesired, because such controls require laborious additional efforts.

Standardization and reproducibility may be hampered, because sera from different animals or in different states of female and other hormone cycles may bear varying properties. In some applications, standardization and reproducibility is, however, of high interest to achieve a well-defined product. In addition, there may be ethical discussions when using high amounts of animal-derived sera, because the use of high contents of serum are obtained from animal bodies, typically from slaughtered animals. Furthermore, serum is usually a big cost driver of cell culture media.

Therefore, there is a need to eliminate the use of animal-derived serum in cell cultivation.

Animal serum is intrinsically complex, the individual components of serum and their concentrations are often not entirely known. The complex nature of animal serum makes it challenging to replace it with alternative components. Moreover, serum contains a multitude of components, suitable for a wide range of cell types and cultivation processes. On the other hand, serum-free media are comparably minimalistic and thus are usually designed for usage with a specific cell line in combination with a particular cultivation process. This means that finding a suitable serum-free medium formulation for an individual process is very challenging.

The cultivated meat industry aims to produce biomass in vitro, particularly by cultivation of animal cells in bioreactors. One of the major limitations in the industry is that cell line-medium combinations that have the properties to efficiently grow biomass in such environments are not available or at least unknown for large scale applications, especially for vertebrate cell lines. One reason for this limitation may be seen in that the cell types typically used for cultivated meat are naturally growing adherent (2-dimensionally) as opposed to in suspension, the latter being preferable for large scale cultivation due to the availability of a third dimension for cell growth.

However, there are reports of large-scale productions of chicken cells (see, e.g., GOOD Meat Inc. (2023); DOSSIER IN SUPPORT OF THE SAFETY OF GOOD MEAT CULTURED CHICKEN AS A HUMAN FOOD INGREDIENT; and Pasitka, L., Wissotsky, G., Ayyash, M. et al. "Empirical economic analysis shows cost-effective continuous manufacturing of cultivated chicken using animal-free medium", Nat Food 2024, 5, 693-702).

Different Serum-free media for cultivation of vertebrate cells have already been described in EP-B 2 601 288 and US 9,593,307, in which animal-derived serum is substituted with purified components tailored to the needs of the individual cell lines and free of animal components. A key medium formulation developed for cultivation of human pluripotent stem cells is "Essential 8", composed of eight components including the basal medium mixture DMEM/F12 (Chen et al., "Chemically defined conditions for human iPS cell derivation and culture", Nat Methods 2011, 8(5), 424-429).

One major problem that arises in attempts to suspension-cultivate cells that normally grow as adherent cultures is the formation of cell aggregates, also known as cell clumping. This phenomenon is typically found when adherent cells are cultivated in suspension conditions, because of the lack of a surface that usually serves as a suitable mimetic of extracellular matrix for such cell lines.

Surfactants or other anti-clumping agents are often used to reduce formation of aggregates and are typical additives to suspension culture media. Aggregate formation is considered one of the biggest obstacles in serum-free culture (Jang M, Scheffold J, Post LM, Cheon H, Bruheim P, "Serum-free cultures of C2C12 cells show different muscle phenotypes which can be estimated by metabolic profiling", Sci Rep. 2022; 12(1), 827).

Accordingly, there is a need for serum-free culture media suitable for cultivation of vertebrate cells in suspension, in which proliferation and viability of the cells are high, but the formation of aggregates is low.

It was now found that suspension cultivation of vertebrate cells, such as those used in the production of cultivated meat, can be performed with high proliferation rates and long-term cell viability and a low rate of cell aggregate formation, when a serum-free culture medium comprising a specific combination of components is used.

Therefore, one aspect of the present invention is a nutrient composition comprising the following components:
a) at least one basal nutrient medium;
b) at least one antioxidant, preferably ascorbic acid or a derivative thereof, more preferably L-ascorbic acid-2-phosphate or a salt thereof;
c) at least one source of selenium, preferably sodium selenite;
d) at least one insulin;
e) at least one transferrin; and
f) at least one growth factor;
g) at least one albumin; and
h) optionally at least one extract, protein isolate and/or hydrolysate of at least one plant or fungus, preferably yeast,
wherein the nutrient composition is free of animal-derived serum.

The at least one basal nutrient medium used in the nutrient composition of the present invention is not particularly limited and may be any basal nutrient medium suitable for cell cultivation.

Typically, the basal nutrient medium comprises a balanced mixture of salts (e.g. sodium, potassium, magnesium, calcium, iron, zinc salts), amino acids (often the 20 proteinogenic amino acids glycine, L-alanine, L-arginine, L-asparagine, L-aspartic acid, L-cysteine, L-glutamine, L-glutamic acid, L-histidine, L-isoleucine, L-leucine, L-lysine, L-methionine, L-phenylalanine, L-proline, L-serine, L-threonine, L-tryptophan, L-tyrosine, L-valine and/or biologically acceptable salts or derivatives thereof), vitamins (such as D-biotin, choline, folic acid, myo-inositol, niacin amide, D-pantothenic acid, pyridoxal, riboflavin, thiamine), and optionally trace elements, fatty acids, carbohydrates (e.g. D-glucose) and further additives (e.g. dispersing agents, shear-stress-reducing agents, surfactants, anti-microbial agents, preservatives, etc.), and serves as the source of nutrients required for the development of living cells.

The basal nutrient medium may e.g. be prepared manually from the respective components, or may be selected from a range of commercially available, ready-to-use basal nutrient media or mixtures thereof.

For example, ready-to-use products suitable as the basal nutrient medium and commercially available from various sources include, but are not limited to, "Ham's F-12 Nutrient Mix" (F12), "Dulbecco's Modified Eagle Medium" (DMEM) and mixtures thereof. Moreover, Basal nutrient media optimized for specific types of cell cultures, available from different suppliers may be used, e.g. basal nutrient media optimized for suspension cultivation of Chinese hamster ovary cells or optimized for suspension cultivation of HEK293 cells.

In some embodiments, the basal nutrient medium comprises DMEM, F12 or a mixture of DMEM and F12 (DMEM/F12). For example, the basal nutrient medium may comprise DMEM/F12, wherein the ratio of DMEM:F12 is in the range of from 1:3 to 3:1, preferably 1:2 to 2:1, more preferably 1:1. Such mixtures are commercially available and can be used directly as a basal nutrient medium in the nutrient composition of the invention.

Components of exemplary commercially available DMEM and F12 and their exemplary mixtures are presented in Table 1 below.

**Table 1 (rounded concentrations given in mmol/L, based on the total volume of the basal nutrient medium; derivatives, salts and hydrates summarized as the respective base compound; inorganic salts not listed)**

| | F12 | DMEM | DMEM:F12 | | | | |
|---|---|---|---|---|---|---|---|
| | | | 1:3 | 1:2 | 1:1 | 2:1 | 3:1 |
| Glycine | 0.1 | 0.4 | 0.175 | 0.2 | 0.25 | 0.3 | 0.325 |
| L-Alanine | 0.1 | 0 | 0.075 | 0.067 | 0.05 | 0.033 | 0.025 |
| L-Arginine | 1.0 | 0.4 | 0.85 | 0.8 | 0.7 | 0.6 | 0.55 |
| L-Asparagine | 0.1 | 0 | 0.075 | 0.067 | 0.05 | 0.03 | 0.025 |
| L-Aspartic acid | 0.1 | 0 | 0.075 | 0.067 | 0.05 | 0.033 | 0.025 |
| L-Cysteine | 0.2 | 0 | 0.15 | 0.13 | 0.1 | 0.067 | 0.05 |
| L-Cystine | 0 | 0.2 | 0.05 | 0.067 | 0.1 | 0.13 | 0.15 |
| L-Glutamic Acid | 0.1 | 0 | 0.075 | 0.067 | 0.05 | 0.033 | 0.025 |
| L-Glutamine | 1 | 4 | 1.75 | 2 | 2.5 | 3 | 3.25 |
| L-Histidine | 0.1 | 0.2 | 0.125 | 0.133 | 0.15 | 0.167 | 0.175 |
| L-Isoleucine | 0.03 | 0.8 | 0.22 | 0.29 | 0.42 | 0.54 | 0.61 |
| L-Leucine | 0.1 | 0.8 | 0.28 | 0.33 | 0.45 | 0.57 | 0.63 |
| L-Lysine | 0.2 | 0.8 | 0.35 | 0.4 | 0.5 | 0.6 | 0.65 |
| L-Methionine | 0.03 | 0.2 | 0.073 | 0.087 | 0.116 | 0.144 | 0.159 |
| L-Phenylalanine | 0.03 | 0.4 | 0.12 | 0.15 | 0.22 | 0.28 | 0.31 |
| L-Proline | 0.3 | 0 | 0.225 | 0.2 | 0.15 | 0.1 | 0.075 |
| L-Serine | 0.1 | 0.4 | 0.175 | 0.2 | 0.25 | 0.3 | 0.325 |
| L-Threonine | 0.1 | 0.8 | 0.28 | 0.33 | 0.45 | 0.57 | 0.62 |
| L-Tryptophan | 0.01 | 0.08 | 0.027 | 0.033 | 0.044 | 0.056 | 0.061 |
| L-Tyrosine | 0.03 | 0.4 | 0.12 | 0.15 | 0.21 | 0.28 | 0.31 |
| L-Valine | 0.1 | 0.8 | 0.28 | 0.33 | 0.45 | 0.57 | 0.63 |
| Biotin | 3×10⁻⁵ | 0 | 2.2×10⁻⁵ | 2×10⁻⁵ | 1.4×10⁻⁵ | 1×10⁻⁵ | 7×10⁻⁶ |
| Choline chloride | 0.1 | 0.03 | 0.082 | 0.076 | 0.064 | 0.052 | 0.046 |
| D-panthotenic acid | 0.001 | 0.008 | 0.0029 | 0.0035 | 0.0047 | 0.0059 | 0.0066 |
| Folic Acid | 0.003 | 0.009 | 0.004 | 0.005 | 0.006 | 0.0070 | 0.008 |
| Niacinamide | 3×10⁻⁴ | 0.03 | 0.008 | 0.011 | 0.017 | 0.022 | 0.025 |
| Pyridoxine | 3×10⁻⁴ | 0.02 | 0.005 | 0.007 | 0.01 | 0.013 | 0.015 |
| Riboflavin | 1×10⁻⁴ | 0.001 | 3×10⁻⁴ | 4×10⁻⁴ | 6×10⁻⁴ | 7×10⁻⁴ | 8×10⁻⁴ |
| Thiamine | 9×10⁻⁴ | 0.01 | 0.0036 | 0.0046 | 0.0064 | 0.0082 | 0.0091 |
| Vitamin B12 | 0.001 | 0 | 8×10⁻⁴ | 7×10⁻⁴ | 5×10⁻⁴ | 3×10⁻⁴ | 3×10⁻⁴ |
| Inositol | 0.1 | 0.04 | 0.085 | 0.08 | 0.07 | 0.06 | 0.055 |
| D-Glucose | 10 | 25 | 13.8 | 15.0 | 17.5 | 20.0 | 21.3 |
| Hypoxanthine | 0.03 | 0 | 0.023 | 0.02 | 0.015 | 0.01 | 0.008 |
| Linoleic Acid | 3×10⁻⁴ | 0 | 2.2×10⁻⁴ | 2×10⁻⁴ | 1.5×10⁻⁴ | 1×10⁻⁴ | 8×10⁻⁵ |
| Lipoic Acid | 0.001 | 0 | 8×10⁻⁴ | 7×10⁻⁴ | 5×10⁻⁴ | 3×10⁻⁴ | 3×10⁻⁴ |
| Putrescine | 0.001 | 0 | 8×10⁻⁴ | 7×10⁻⁴ | 5×10⁻⁴ | 3×10⁻⁴ | 3×10⁻⁴ |
| Sodium Pyruvate | 1 | 0 | 0.75 | 0.67 | 0.5 | 0.33 | 0.25 |
| Thymidine | 0.003 | 0 | 0.0022 | 0.0019 | 0.0015 | 0.001 | 7×10⁻⁴ |

The composition of DMEM, F12 and mixtures thereof may vary between different commercial products and suppliers. For example, different derivatives, salts and hydrates of amino acids and/or vitamins may be used, and different amounts of inorganic salts are often used. Often, the inorganic salts will include chloride, sulfate (mono- and/or dibasic), carbonate (mono- and/or dibasic), phosphate (mono-, di- and/or tribasic) and/or nitrate salts of sodium, potassium, magnesium, calcium, iron, zinc and/or copper in varying amounts. For example, the inorganic salts often include calcium chloride, cupric sulfate, ferric nitrate, ferric sulfate, magnesium chloride, magnesium sulfate, potassium chloride, sodium bicarbonate, sodium chloride, sodium phosphate (mono- and/or dibasic), and zinc sulfate. For example, DMEM often contains 1.5 to 2 mmol/L (e.g. 1.8 mmol/L) of calcium chloride, 1×10⁻⁵ to 5×10⁻⁵ mmol/L (e.g. 2.5×10⁻⁴ mmol/L) of ferric nitrate, 0.5 to 1.5 mmol/L (e.g. 0.8 mmol/L) of magnesium sulfate, 5 to 6 mmol/L (e.g. 5.3 mmol/L) of potassium chloride, 40 to 50 mmol/L (e.g. 44 mmol/L) of sodium bicarbonate, 100 to 150 mmol/L (e.g. 110 mmol/L) of sodium chloride, and 0.5 to 1.5 mmol/L (e.g. 0.9 mmol/L) of sodium phosphate (often monobasic).

For example, F12 often contains 0.1 to 0.5 mmol/L (e.g. 0.03 mmol/L) of calcium chloride; 0.5×10⁵ to 1.5×10⁵ mmol/L (e.g. 1×10⁵ mmol/L) of cupric sulfate, 0.001 to 0.005 mmol/L (e.g. 0.003 mmol/L) of ferric sulfate, 0.3 to 1 mmol/L (e.g. 0.6 mmol/L) of magnesium chloride, 1 to 5 mmol/L (e.g. 3 mmol/L) of potassium chloride, 10 to 20 mmol/L (e.g. 14 mmol/L) of sodium bicarbonate, 100 to 150 mmol/L (e.g. 130 mmol/L) of sodium chloride, 0.5 to 1.5 mmol/L (e.g. 1 mmol/L) of sodium phosphate (often dibasic), and 0.001 to 0.005 mmol/L (e.g. 0.003 mmol/L) of zinc sulfate.

In some embodiments, the basal nutrient medium has an osmolality in the range of from 250 to 400 mOsm/kg H₂O, preferably 280 to 350 mOsm/kg H₂O, more preferably from 300 to 340 mOsm/kg H₂O, and a pH in the range of from 6.5 to 8.0, preferably from 6.8 to 7.8, more preferably from 7.0 to 7.6. For example, these properties may be obtained by dissolving the components in a buffer and/or adjusting the amount of salts, such as sodium bicarbonate, in the nutrient medium. For example, a 4-(2-hydroxyethyl)-1-piperazineethanesulfonic acid (HEPES) buffer may be present in the basal nutrient medium, to obtain a desired pH value. The pH value may be controlled e.g. by a biologically acceptable indicator, such as phenol red.

The basal nutrient mixture may be used in the nutrient composition directly, or may be part of one or more complex mixtures comprising one or more of the at least one antioxidant, the at least one source of selenium, the at least one insulin, the at least one transferrin, the at least one growth factor, the at least one albumin, and/or the optional at least one extract, protein isolate and/or hydrolysate of at least one plant or fungus.

For example, commercially available products useful for preparing the nutrient composition of the invention are, among others, Essential 6^{™} (Gibco^{™}), which comprises a 1:1 DMEM/F12 basal nutrient medium, L-ascorbic acid-2-phosphate magnesium salt as an antioxidant, sodium selenite, insulin and transferrin; Essential 8^{™}(Gibco^{™}) which comprises a 1:1 DMEM/F12 basal nutrient medium, L-ascorbic acid-2-phosphate magnesium salt as an antioxidant, sodium selenite, insulin, transferrin and growth factors FGF2 and TGFβ1; or "CHO medium serumfrei" (Bio&Sell) which comprises a basal nutrient medium optimized for suspension cultivation of Chinese Hamster Ovary cells, and comprises a yeast extract, and is enriched with stabilized L-glutamine, HEPES buffer, iron citrate, and a dispersing agent.

The at least one antioxidant used in the nutrient composition of the present invention is not particularly limited and may be any biologically acceptable antioxidant suitable for cell cultivation. Preferably, the antioxidant is at least one antioxidant that is typically found in living organisms.

Examples of suitable antioxidants include ascorbic acid, glutathione, lipoic acid, uric acid, carotenes, α-tocopherol, ubiquinol, catalase, superoxide dismutase, peroxiredoxins, thioredoxins, and derivatives thereof, such as stabilized derivatives. Preferably, the nutrient composition comprises one or more of these antioxidants, more preferably one or more, preferably all antioxidants selected from ascorbic acid and derivatives thereof, catalase, glutathione, superoxide dismutase, DL-α-tocopherol and derivatives thereof (such as DL-α-tocopherol acetate). More preferably, the one or more antioxidants comprise at least ascorbic acid or a derivative thereof, preferably an L-ascorbic acid-2-phosphate salt, more preferably L-ascorbic acid-2-phosphate magnesium salt.

Preferably, the amount of antioxidants, preferably the amount of ascorbic acid and derivatives thereof, in the nutrient composition of the invention is in the range of from 3 to 200 mg/L, more preferably from 5 to 100 mg/L, more preferably from 6 to 64 mg/L, more preferably from 8 to 40 mg/L, more preferably 8 to 20 mg/L, based on the total volume of the nutrient composition.

The at least one source of selenium used in the nutrient composition of the present invention is not particularly limited and may be any compound that provides selenium in a biologically accessible form. For example, the source of selenium may be sodium selenite or sodium selenate. Preferably, the source of selenium is sodium selenite.

The amount of the at least one source of selenium, in particular sodium selenite, is preferably in the range of from 0.5 to 40 µg/L, more preferably from 1 to 25 µg/L, more preferably from 1.5 to 15 µg/L, more preferably from 1.75 to 10 µg/L, based on the total volume of the nutrient composition.

The at least one insulin used in the nutrient composition of the present invention is not particularly limited and may be an insulin of any origin.

For example, the insulin may be obtained from any vertebrate species such as mammals (e.g., sheep, cow/beef, donkey, goat, pig, buffalo, deer, horse, camel, rabbit, etc.), birds (e.g., duck, chicken, goose, turkey, goose, pigeon, fowl, etc.), reptiles (e.g., alligator, crocodile, snake etc.), fish (e.g., salmon), or may be recombinant insulin. When the insulin is obtained from a vertebrate species, it preferably stems from the same vertebrate species as the cells being cultivated.

However, it is desirable to keep the use of animal components to a minimum. Therefore, the insulin is preferably recombinant insulin (including human recombinant insulin). When the insulin is recombinant insulin, it can be the recombinant version of the insulin stemming from the same vertebrate species as the cells being cultivated. Alternatively, the recombinant insulin may be recombinant human insulin, which for years is produced on a larger scale for medicinal purposes, and is therefore associated with lower costs due to high availability.

The amount of insulin, preferably recombinant vertebrate insulin, e.g. recombinant human insulin, is preferably in the range of from 0.5 to 100 mg/L, more preferably from 1.8 to 50 mg/L, more preferably from 2 to 20 mg/L, more preferably from 2.4 to 10 mg/L, based on the total volume of the nutrient composition.

The at least one transferrin is not particularly limited and may be a transferrin of any origin. For example, the transferrin may be obtained from any vertebrate species such as mammals (e.g., sheep, cow/beef, donkey, goat, pig, buffalo, deer, horse, camel, rabbit, etc.) birds (e.g., duck, chicken, goose, turkey, goose, pigeon, fowl, etc.), reptiles (e.g., alligator, crocodile, snake etc.), fish (e.g., salmon), or may be recombinant transferrin. When the transferrin is obtained from a vertebrate species, it preferably stems from the same vertebrate species as the cells being cultivated.

However, it is desirable to keep the use of animal components to a minimum.

Therefore, the transferrin is preferably recombinant transferrin (including human recombinant transferrin). When the transferrin is recombinant, it can be the recombinant version of the transferrin stemming from the same vertebrate species as the cells being cultivated. Alternatively, the recombinant transferrin may be recombinant human transferrin, which is produced on a larger scale for medicinal purposes, and is therefore associated with lower costs due to high availability. Moreover, the transferrin may be iron-loaded transferrin (holo-transferrin), or may be iron-free-transferrin (apo-transferrin), depending on the iron concentration in the nutrient composition (e.g. as part of the basal nutrient medium). Preferably, the transferrin is a holo-transferrin, an apo-transferrin, or a combination of holo-transferrin and apo-transferrin.

The amount of transferrin, preferably recombinant vertebrate transferrin, e.g. recombinant human transferrin, is preferably in the range of from 0.5 to 20 mg/L, more preferably from 1 to 15 mg/L, more preferably from 1.2 to 9 mg/L, more preferably from 1.3 to 5 mg/L, based on the total volume of the nutrient composition.

The at least one growth factor used in the nutrient composition of the present invention is not particularly limited and may be any compound that is known to stimulate growth factor receptors of the cells to be cultivated.

Preferably, the growth factor is selected from fibroblast growth factor receptor (FGFR) agonists, transforming growth factor receptor (TGFR) agonists and combinations thereof. More preferably, the growth factor is at least one FGFR agonist or a combination thereof with at least one TGFR agonist. Preferably, the FGFR agonist is selected from FGF2 (e.g. human FGF-basic recombinant protein) or a modified variant/mutant thereof, such as the stabilized variant FGF2-G3 (by Defined Bioscience), which is commercially available. Preferably, the TGFR agonist is a TGFβ receptor agonist, more preferably a TGFβ receptor I agonist. More preferably, the TGFR agonist is TGFβ1 or a modified variant/mutant thereof.

The growth factors may be obtained from any vertebrate species such as mammals (e.g., sheep, cow/beef, donkey, goat, pig, buffalo, deer, horse, camel, rabbit, etc.), birds (poultry/avian, e.g., duck, chicken, goose, turkey, goose, pigeon, fowl, etc.), reptiles (e.g., alligator, crocodile, snake etc.), fish (e.g., salmon), or may be recombinant growth factors. When the growth factors are obtained from a vertebrate species, they preferably stem from the same vertebrate species as the cells being cultivated.

However, it is desirable to keep the use of animal components to a minimum. Therefore, the growth factors are preferably recombinant (including human recombinant growth factors).

When the growth factors are recombinant, they can be the recombinant versions of the growth factors stemming from the same vertebrate species as the cells being cultivated. Alternatively, the recombinant growth factors may be recombinant human growth factors.

The total amount of growth factors is preferably in the range of from 2 to 200 µg/L, preferably from 4 to 100 µg/L, based on the total volume of the nutrient composition. More preferably, the nutrient composition of the invention comprises from 4 to 100 µg/L, preferably from 8 to 80 µg/L, more preferably from 10 to 70 µg/L, more preferably from 30 to 40 µg/L, based on the total volume of the nutrient composition, of at least one fibroblast growth factor receptor agonist, preferably modified or unmodified FGF2, and optionally from 0.1 to 4 µg/L, preferably from 0.2 to 3 µg/L, more preferably from 0.25 to 2 µg/L, more preferably from 0.25 to 1 µg/L, based on the total volume of the nutrient composition, of a transforming growth factor beta receptor agonist, preferably modified or unmodified TGFβ1.

The at least one albumin used in the nutrient composition of the present invention is not particularly limited and may be an albumin of any origin. For example, the albumin may be obtained from plants (e.g. wheat), vertebrate species such as mammals (e.g., sheep, cow/beef, donkey, goat, pig, buffalo, deer, horse, camel, rabbit, etc.), birds (e.g., duck, chicken, goose, turkey, goose, pigeon, fowl, etc.), reptiles (e.g., alligator, crocodile, snake etc.), fish (e.g., salmon), or may be recombinant albumin. When the albumin is obtained from a vertebrate species, it preferably stems from the same vertebrate species as the cells being cultivated.

However, it is desirable to keep the use of animal components to a minimum. Therefore, the albumin is preferably recombinant albumin (including human recombinant albumin). When the albumin is recombinant albumin, it can be the recombinant version of the albumin stemming from the same vertebrate species as the cells being cultivated. Alternatively, the recombinant albumin may be recombinant human albumin, which is produced on a larger scale for medicinal purposes, and is therefore associated with lower costs due to high availability.

The amount of albumin, preferably recombinant vertebrate albumin, e.g. recombinant human albumin, is preferably in the range of from 25 to 1000 mg/L, more preferably 50 to 800 mg/L, more preferably 100 to 500 mg/L, more preferably 150 to 350 mg/L, based on the total volume of the nutrient composition.

It has been found that the presence of albumin, especially in the specified concentration ranges, substantially reduces the formation of cell aggregates in suspension cultures, leading to fewer and smaller cell aggregates, when the nutrient composition of the invention is used as culture medium, compared with compositions lacking albumin. In addition, it has been found that albumin positively affects cell proliferation and cell viability.

Preferably, the nutrient composition of the invention comprises the at least one extract, protein isolate and/or hydrolysate of at least one plant or fungus. The at least one extract, protein isolate and/or hydrolysate of at least one plant or fungus used is not particularly limited and may originate from any plant or fungus, provided the extract, protein isolate and/or hydrolysate is free of components that are toxic or growth-inhibitory for the cells to be cultivated at the concentrations used in the nutrient composition.

For example, the extract, protein isolate and/or hydrolysate may be obtained from plants such as soy, rapeseed, cottonseed, wheat and algae (including microalgae). Preferably, the extract, protein isolate and/or hydrolysate is a fungal extract, protein isolate and/or hydrolysate. More preferably, it is a yeast extract, protein isolate and/or hydrolysate. More preferably, it is a yeast extract, e.g. yeast extract that has been subjected to ultrafiltration.

The amount of the at least one extract, protein isolate and/or hydrolysate of at least one plant or fungus is preferably in the range of from 10 to 5000 mg/L, preferably from 25 to 2000 mg/L, more preferably from 50 to 1500 mg/L, more preferably from 100 to 1000 mg/L, based on the total volume of the nutrient composition.

While compositions of the invention without the at least one extract, protein isolate and/or hydrolysate of at least one plant or fungus already show beneficial properties in terms of cell proliferation, cell viability and low aggregate formation, it has been found that the presence of at least one extract, protein isolate and/or hydrolysate of at least one plant or fungus, in particular yeast extract, especially in the specified concentration ranges, further substantially enhances cell proliferation and viability, when the nutrient composition of the invention is used as culture medium, compared with compositions lacking an extract, protein isolate and/or hydrolysate of at least one plant or fungus.

This enhancement is particularly pronounced in suspension cultures. In addition, it has been found that the transfer of adherent cell cultures into suspension is facilitated, when at least one extract, protein isolate and/or hydrolysate of at least one plant or fungus, in particular yeast extract is present.

In addition to the above components, it can be beneficial for the nutrient composition of the invention to contain additional components, e.g. further biologically active ingredients or additives for modification of the physical properties of the cell culture or of the nutrient composition. For example, the nutrient composition may contain one or more components selected from enzymes, co-enzymes, hormones (e.g. steroids), nucleosides, nucleotides, dispersing agents (e.g. surfactants and polymers such as polysaccharides, polyamines, polyimines and polyglycols), rheology-modifying agents, thixotropic agents, lubricating agents, shear stress reducing agents, additional anti-clumping agents, pH modifying agents (such as acids, bases and/or buffers), anti-microbial agents and preservatives, provided these are not toxic or growth-inhibitory to the cells to be cultivated.

When the nutrient composition is used for suspension cultures, it preferably comprises at least one dispersing agent, more preferably at least one polyglycol, e.g. a polyoxyethylene-polyoxypropylene block copolymer such as those distributed under the trade name Pluronic^{®}.

In some embodiments, the nutrient composition of the invention comprises, based on the total volume of the nutrient composition:
from 3 to 200 mg/L, preferably from 6 to 64 mg/L, more preferably from 8 to 40 mg/L, more preferably from 8 to 20 mg/L, of the at least one antioxidant, preferably ascorbic acid or a derivative thereof, more preferably L-ascorbic acid-2-phosphate or a salt thereof, more preferably L-ascorbic acid-2-phosphate magnesium salt;
from 0.5 to 40 µg/L, preferably from 1 to 25 µg/L, more preferably from 1.5 to 15 µg/L, more preferably from 1.75 to 10 µg/L, of sodium selenite;
from 0.5 to 100 mg/L, preferably from 1.8 to 50 mg/L, more preferably from 2 to 20 mg/L, more preferably from 2.4 to 10 mg/L, of insulin;
from 0.5 to 20 mg/L, preferably from 1 to 15 mg/L, more preferably from 1.2 to 9 mg/L, more preferably from 1.3 to 5 mg/L, of transferrin;
from 4 to 100 µg/L, preferably from 8 to 80 µg/L, more preferably from 10 to 70 µg/L, more preferably from 30 to 40 µg/L, of a fibroblast growth factor receptor agonist, preferably modified or unmodified FGF2;
optionally from 0.1 to 4 µg/L, preferably from 0.2 to 3 µg/L, more preferably from 0.25 to 2 µg/L, more preferably from 0.25 to 1 µg/L, of a transforming growth factor beta receptor agonist, preferably modified or unmodified TGFβ1;
from 25 to 1000 mg/L, preferably 50 to 800 mg/L, more preferably 100 to 500 mg/L, more preferably 150 to 350 mg/L, of albumin; and
optionally from 10 to 5000 mg/L, preferably from 25 to 2000 mg/L, more preferably from 50 to 1500 mg/L, more preferably from 100 to 1000 mg/L, of yeast extract.

If the nutrient composition does not contain an extract, protein isolate and/or hydrolysate of at least one plant or fungus, the nutrient composition preferably comprises, based on the total volume of the nutrient composition:
from 25 to 45 mg/L, preferably from 27 to 40 mg/L, more preferably from 30 to 35 mg/L, of ascorbic acid or a derivative thereof;
from 0.9 to 1.6 mg/L, preferably from 1 to 1.5 mg/L, more preferably from 1.2 to 1.3 mg/L, of at least one catalase;
from 45 to 80 mg/L, preferably from 50 to 75 mg/L, more preferably from 55 to 70 mg/L, of glutathion;
from 0.9 to 1.6 mg/L, preferably from 1 to 1.5 mg/L, more preferably from 1.2 to 1.3 mg/L, of at least one superoxide dismutase;
from 0.35 to 0.65 mg/L, preferably from 0.4 to 0.6 mg/L, more preferably from 0.45 to 0.55 mg/L, of tocopherol; and
from 0.35 to 0.65 mg/L, preferably from 0.4 to 0.6 mg/L, more preferably from 0.45 to 0.55 mg/L, of tocopherol acetate;
from 12 to 15 µg/L, preferably from 13 to 14 µg/L, more preferably from 13.2 to 13.5 µg/L of sodium selenite;
from 9 to 15 mg/L, preferably from 10 to 13.5 mg/L, more preferably from 10.5 to 12 mg/L of at least one insulin;
from 7.3 to 9.4 mg/L, preferably from 7.5 to 8.7 mg/L, more preferably from 7.6 to 8.2 mg/L of at least one transferrin;
from 30 to 70 µg/L, preferably from 40 to 60 µg/L, more preferably from 45 to 55 µg/L of FGF2;
from 0.5 to 1.5 µg/L, preferably from 0.8 to 1.2 µg/L, more preferably from 0.9 to 1.1 µg/L of TGFβ1;
from 500 to 1200 mg/L, preferably from 600 to 1000 mg/L, more preferably from 750 to 850 mg/L of at least one albumin;
from 5 to 15 µg/L, preferably from 7 to 13 µg/L, more preferably from 9 to 11 µg/L, of corticosterone;
from 2.2 to 4.1 µg/L, preferably from 2.5 to 3.8 µg/L, more preferably from 2.8 to 3.5 µg/L, of progesterone;
from 35 to 65 µg/L, preferably from 40 to 60 µg/L, more preferably from 45 to 55 µg/L, of retinol acetate;
from 0.35 to 0.65 mg/L, preferably from 0.5 to 0.6 mg/L, more preferably from 0.45 to 0.55 mg/L, of linolenic acid;
from 0.35 to 0.65 mg/L, preferably from 0.5 to 0.6 mg/L, more preferably from 0.45 to 0.55 mg/L, of linoleic acid;
from 0.35 to 0.65 mg/L, preferably from 0.5 to 0.6 mg/L, more preferably from 0.45 to 0.55 mg/L, of pipecolic acid;
from 0.5 to 1.5 µg/L, preferably 0.7 to 1.3 µg/L, more preferably 0.9 to 1.1 µg/L, of triiodothyronine;
from 0.35 to 0.65 µg/L, preferably from 0.5 to 0.6 µg/L, more preferably from 0.45 to 0.55 µg/L, of ethanolamine;
from 5 to 12 mg/L, preferably from 6 to 10 mg/L, more preferably from 7 to 9 mg/L, of putrescine;
from 5 to 10 mg/L, preferably from 6 to 9 mg/L, more preferably from 7 to 8 mg/L, of galactose;
from 0.5 to 1.5 mg/L, preferably from 0.7 to 1.3 mg/L, more preferably from 0.8 to 1.1 mg/L, of L-carnitine; and
from 0.8 to 1.8 mg/L, preferably from 1 to 1.5 mg/L, more preferably from 1.2 to 1.3 mg/L, of biotin.

Preferably, this nutrient composition not containing an extract, protein isolate and/or hydrolysate of at least one plant or fungus comprises DMEM/F12 (e.g. a 3:1 to 1:3 mixture, more preferably 2:1 to 1:2 mixture, more preferably 1:1 mixture) as the basal nutrient medium.

This nutrient composition not containing an extract, protein isolate and/or hydrolysate of at least one plant or fungus, is very well suited for cultivating vertebrate cells in adherent cultures, and leads to substantial improvements in terms of cell viability and cell proliferation, compared with known nutrient compositions such as those described by Kolkmann et al. (Frontiers in Bioengineering and Biotechnology 2022, 10, Article 895289, 1-15) or Tan et al. (Cytotherapy 2015, 17, 1152-1165).

Moreover, this nutrient composition not containing an extract, protein isolate and/or hydrolysate of at least one plant or fungus, can be conveniently used for the preparation of the nutrient composition containing an extract, protein isolate and/or hydrolysate of at least one plant or fungus. For example, the nutrient composition may be mixed with an extract, protein isolate and/or hydrolysate of at least one plant or fungus directly, or may be mixed with a mixture comprising a basal nutrient medium and the extract, protein isolate and/or hydrolysate of at least one plant or fungus.

Preferably, the nutrient composition containing an extract, protein isolate and/or hydrolysate of at least one plant or fungus is prepared by mixing this nutrient composition not containing an extract, protein isolate and/or hydrolysate of at least one plant or fungus with a mixture comprising a basal nutrient medium and an extract, protein isolate and/or hydrolysate of at least one plant or fungus, preferably yeast extract.

An aspect of the present invention is therefore a method for the preparation of the nutrient composition of the invention comprising at least one extract, protein isolate and/or hydrolysate of at least one plant or fungus, preferably yeast extract, wherein the method comprises the steps:
A1) providing the composition of the invention not containing an extract, protein isolate and/or hydrolysate of at least one plant or fungus; and
B1) mixing the composition provided in step A1) with at least one extract, protein isolate and/or hydrolysate of at least one plant or fungus or with at least one composition comprising a basal nutrient medium and at least one extract, protein isolate and/or hydrolysate of at least one plant or fungus, preferably yeast extract. Preferably, the composition of the invention not containing an extract, protein isolate and/or hydrolysate of at least one plant or fungus is mixed with at least one composition comprising a basal nutrient medium and at least one extract, protein isolate and/or hydrolysate of at least one plant or fungus, preferably yeast extract, at a volume ratio (composition of the invention : composition comprising the extract, protein isolate and/or hydrolysate) in the range from 5:95 to 70:30, preferably from 10:90 to 60:40, more preferably from 15:85 to 55:45, more preferably from 25:75 to 50:50.

Another aspect of the invention is a method for the preparation of a nutrient composition, comprising the following steps:
A) providing at least one basal nutrient medium, preferably comprising DMEM/F12, at least one culture medium for cell cultivation in suspension, or a mixture thereof;
B) adjusting therein the amount of at least one antioxidant to a concentration of from 3 to 200 mg/L, preferably from 6 to 64 mg/L, more preferably from 8 to 40 mg/L, more preferably from 8 to 20 mg/L;
C) adjusting therein the amount of sodium selenite to a concentration of from 0.5 to 40 µg/L, preferably from 1 to 25 µg/L, more preferably from 1.5 to 15 µg/L, more preferably from 1.75 to 10 µg/L;
D) adjusting therein the amount of insulin to a concentration of from 1 to 100 mg/L, preferably from 1.8 to 50 mg/L, more preferably from 2 to 20 mg/L, more preferably from 2.4 to 10 mg/L;
E) adjusting therein the amount of transferrin to a concentration of from 0.5 to 20 mg/L, preferably from 1 to 15 mg/L, more preferably from 1.2 to 9 mg/L, more preferably from 1.3 to 5 mg/L; and
F) adjusting therein the amount of at least one fibroblast growth factor receptor agonist, preferably unmodified or modified FGF2 to a concentration of from 4 to 100 µg/L, preferably from 8 to 80 µg/L, more preferably from 10 to 70 µg/L, more preferably from 30 to 40 µg/L;
G) optionally adjusting therein the amount of at least one transforming growth factor beta receptor agonist, preferably modified or unmodified TGFβ1 to a concentration of from 0.1 to 4 µg/L, preferably from 0.2 to 3 µg/L, more preferably from 0.25 to 2 µg/L, more preferably from 0.25 to 1 µg/L; and
H) adjusting therein the amount of albumin, preferably plant albumin or recombinant albumin, to a concentration of from 25 to 1000 mg/L, preferably 50 to 800 mg/L, more preferably 100 to 500 mg/L, more preferably 150 to 350 mg/L; and/or
optionally adjusting therein the amount of at least one extract, protein isolate and/or hydrolysate of at least one plant or fungus, preferably yeast extract, to a concentration of from 10 to 5000 mg/L, preferably from 25 to 2000 mg/L, more preferably from 50 to 1500 mg/L, more preferably from 100 to 1000 mg/L.

Preferably, the method of the invention is for the preparation of the nutrient composition as described above, and the individual components thereof are those described above.

The step A) of providing the at least one basal nutrient medium may be carried out by any suitable means. For example, the basal nutrient medium may be provided by mixing the individual ingredients thereof in a suitable liquid, typically an aqueous solution. Alternatively, a ready-to use, (e.g. commercially available) basal nutrient medium such as DMEM/F12 or a culture medium optimized for suspension cultures can be used directly as the basal nutrient medium.

The at least one basal nutrient medium may also be provided as part of one or more complex mixtures comprising one or more of the other components described above.

For example, the at least one basal nutrient medium or a portion thereof may be provided as part of a mixture comprising the basal nutrient medium and one or more, preferably all of the components selected from at least one antioxidant, at least one source of selenium, at least one insulin, at least one transferrin, and optionally at least one growth factor, as is the case in certain commercial products such as Essential 8^{™} (Gibco^{™}; comprising 1:1 DMEM/F12 and L-ascorbic acid-2-phosphate magnesium salt (64 mg/L), sodium selenite (14 µg/L), insulin (19.4 mg/L), transferrin (10.7 mg/L), FGF2 (100 µg/L), TGFβ1 (2 µg/L) and NaHCO₃ (543 mg/L), as disclosed in Chen et al., "Chemically defined conditions for human iPS cell derivation and culture", Nat. Methods 2011, 8(5), 424-429) and Essential 6^{™} (Gibco^{™}; similar composition as Essential 8^{™}, but without FGF2 and TGFβ1).

Similarly, the at least one basal nutrient medium or a portion thereof may be provided as part of a mixture comprising the basal nutrient medium and at least one extract, protein isolate and/or hydrolysate of at least one plant or fungus, as is the case in certain commercial products such as "CHO medium serumfrei" (Bio&Sell), which comprises a basal nutrient medium and yeast extract.

Steps B) to H) of adjusting the amounts of individual components in the basal nutrient medium may be carried out by any suitable means. For example, if in step A) the basal nutrient medium was provided by mixing the individual ingredients thereof, or a ready-to-use basal nutrient medium is provided, the adjustments in steps B) to H) may be carried out by adding the respective ingredients to the basal nutrient medium until the desired concentration is reached.

In contrast, if in step A) the at least one basal nutrient medium is be provided as part of one or more complex mixtures comprising one or more of the other components described above, the adjustment of the amounts in steps B) to H) may be carried out by selecting an appropriate amount of a complex mixture, and optionally diluting the complex mixture with more basal nutrient medium and optionally adding any missing ingredients to the complex mixture, or by mixing two or more complex mixtures in a ratio that leads to the desired concentrations and optionally adding any missing ingredients to the complex mixture.

A ready-to-use mixture of one or more ingredients may also be used for adjustment of the amounts in steps B) to H). For example, a commercially available ready-to-use mixture useful for adjusting the amounts of antioxidants, sodium selenite, insulin, transferrin and albumin in the method of the invention is B-27^{™} (Gibco^{™}) serum-free supplement (50X) liquid, which comprises bovine serum albumin (BSA), antioxidants (catalase, glutathione, superoxide dismutase, tocopherol, tocopherol acetate), sodium selenite, human recombinant insulin and human transferrin.

In some embodiments of the invention, the basal nutrient medium is provided in step A) as part of a complex mixture comprising a basal nutrient medium, at least one antioxidant, at least one source of selenium, at least one insulin, at least one transferrin, and as part of a complex mixture comprising a basal nutrient medium and at least one extract, protein isolate and/or hydrolysate of at least one plant or fungus, and optionally further basal nutrient medium, and the amounts of antioxidant, source of selenium, insulin, transferrin, and extract, protein isolate and/or hydrolysate of at least one plant or fungus in steps B) to H) are adjusted by selecting an appropriate ratio of the two complex mixtures and optionally further basal nutrient medium, and by adding thereto growth factors and albumin or a mixture of either one or both with other ingredients.

In other embodiments of the invention, the basal nutrient medium is provided in step A) as part of a complex mixture comprising a basal nutrient medium and at least one extract, protein isolate and/or hydrolysate of at least one plant or fungus, optionally diluted with further basal nutrient medium, and the amounts in steps B) to H) are adjusted by adding thereto growth factors and a ready-to-use mixture of antioxidants, sodium selenite, insulin, transferrin and albumin.

In yet other embodiments of the invention, the basal nutrient medium is provided in step A) as a ready to use mixture, or prepared by mixing, and the amounts in steps B) to H) are adjusted by adding the respective amounts of the ingredients to the basal nutrient medium.

For example, the nutrient composition may be prepared by mixing the commercially available products Essential 6^{™}, "CHO medium serumfrei", B-27^{™} and optionally 1:1 DMEM/F12, and adding thereto FGF2 or a mixture thereof with TGFβ1.

Alternatively, the nutrient composition may be prepared by mixing the commercially available products Essential 6^{™}, "CHO medium serumfrei" and optionally 1:1 DMEM/F12, and adding thereto albumin (e.g. recombinant human albumin) FGF2 or a mixture thereof with TGFβ1. Alternatively, the nutrient composition may be prepared by mixing the commercially available products "CHO medium serumfrei", B-27^{™} and optionally 1:1 DMEM/F12, and adding thereto FGF2 or a mixture thereof with TGFβ1. Alternatively, the nutrient composition may be prepared by mixing the commercially available products Essential 8^{™}, "CHO medium serumfrei", B-27^{™} or albumin, and optionally 1:1 DMEM/F12.

Alternatively, the nutrient composition may be prepared by mixing the commercially available products B-27^{™} and 1:1 DMEM/F12, and adding thereto FGF2 or a mixture thereof with TGFβ1 and preferably a yeast extract suspension. Alternatively, the nutrient composition may be prepared by providing a basal nutrient medium such as 1:1 DMEM/F12 and adding thereto at least one antioxidant, at least one selenium source, at least one insulin, at least one transferrin, at least one albumin, at least one extract, protein isolate and/or hydrolysate of at least one plant or fungus, and at least one growth factor.

Alternatively, the nutrient composition may be prepared by preparing an aqueous mixture of salts, amino acids, vitamins, fatty acids, carbohydrates (e.g. D-glucose), at least one antioxidant, at least one selenium source, at least one insulin, at least one transferrin, at least one albumin, optionally at least one extract, protein isolate and/or hydrolysate of at least one plant or fungus, and at least one growth factor and optionally further additives. Further ways of carrying out the method according to the invention will be apparent to those skilled in the art.

Another aspect of the present invention is a cell culture comprising non-human vertebrate-derived cells and the nutrient composition as described above, or prepared by the method described above. Yet another aspect of the present invention is the use of the nutrient composition as described above, or prepared by the method described above, for the cultivation of non-human vertebrate-derived cells.

It has been found that, while intended for use in suspension cultures, the nutrient composition of the invention is also suitable for cultivation of cells in other types of cultures, e.g. adherent cultures.

The cell culture of the invention therefore may be any type of cell culture, e.g. an adherent culture, or a suspension culture. Preferably, the cell culture is a suspension culture.

The non-human vertebrate cells used in the cell culture according to the present invention may originate from any non-human mammal (e.g., sheep, cow/beef, donkey, goat, pig, buffalo, deer, horse, camel, rabbit, etc.), bird (e.g., duck, chicken, goose, turkey, goose, pigeon, fowl, etc.), reptile (e.g., alligator, crocodile, snake etc.), or fish (e.g., salmon).

Preferably, the cells are non-human mammalian cells or avian cells. More preferably, the cells are bovine cells, porcine cells or chicken cells. In one embodiment, cells of interest may originate from a ruminant such as, e.g., may be selected from the group consisting of cow, sheep, goat, deer, and buffalo cells. In one embodiment, cells of interest may originate from a non-ruminant such as, e.g., may be pig cells. In one embodiment, cells of interest may originate from a pseudo-ruminant such as, e.g., may be selected from the group consisting of horse, camel, and rabbit cells. The cells of interest may originate from any non-human vertebrate species ("donor").

In one embodiment, cells of interest may be primary cells originating from a mammal (mammalian animal), from a bird species, from a reptile or a fish. In another embodiment, cells of interest may be cells from a permanent mammal, bird, reptile or fish cell culture. In one embodiment, the cells used in the context of the present invention are primary mammalian cells (also: mammal cells). In one embodiment, the cells used in the context of the present invention are from a mammalian cell culture (also: mammal cell culture).

Moreover, the cells used in the cell culture according to the present invention may be any type of cells, including, but not limited to, muscle-tissue-derived cells, fat-tissue-derived cells, connective-tissue-derived cells, kidney-tissue-derived cells, liver-tissue-derived cells and other organ-tissue-derived cells. Preferably, the cells are selected from muscle-tissue-derived cells, fat-tissue-derived cells and connective-tissue-derived cells. More preferably, the cells are muscle-tissue-derived cells.

Another aspect of the invention is a method for the cultivation of non-human vertebrate-derived cells, comprising the following steps:
1) introducing vertebrate-derived cells into the nutrient composition as described above or prepared by the method described above, in order to obtain a cell culture;
2) optionally agitating the cell culture, preferably by stirring, shaking, rocking, and/or inverting;
3) incubating the cell culture;
4) optionally exchanging the nutrient composition in the cell culture with fresh nutrient composition as described above or prepared by the method described above;
5) optionally feeding additional nutrient composition or individual components thereof to the cell culture, depending on consumption thereof.

It will be understood by those skilled in the art that the optional agitation of step 2) and the optional exchange or feeding of steps 4) and 5) may be carried out simultaneously with the incubation step 3), and are not necessarily consecutive.

Prior to step 1), the method may involve further steps, in which the vertebrate-derived cells are prepared for the cultivation. For example, the cells may be subjected to one or more steps selected from cell immortalization, harvesting from a donor organism, harvesting from another cell culture, filtration, dialysis, centrifugation, rinsing and/or adaptation to a nutrient composition.

Cells may optionally be stored as a cell stock at any condition usable for such purpose such as in a refrigerator (e.g. at 1 to 10°C, e.g., (approximately) 4°C), in a freezer (e.g. at -30 to 0°C, e.g., (approximately) -20°C), in a high-performance freezer (e.g. at -90 to -70°C, e.g., (approximately) -80°C), in an ultra low temperature freezer (e.g. at approximately -160 to -140 °C, e.g. (approximately) -150 °C) or in liquid nitrogen (N₂). Optionally, a frozen stock may or may not contain dimethyl sulfoxide (DMSO).

The introduction of the cells into the nutrient composition may be carried out in any suitable reactor, such as a bioreactor, flask, dish or other vessel.

For example, bioreactors may be used for cultivating the cells. This may be any type of bioreactor. For instance, a suitable bioreactor may be a stirred tank bioreactor, a wave bioreactor, an airlift bioreactor, a packed bed bioreactor, a vertical wheel bioreactor, or a hollow fiber bioreactor.

The method of the present invention may be scaled up to a desired degree. A bio reactor may have any size such as a volume of below 10 L, of from 10 to 50 L, of from 20 to 100 L, of from 50 to 250 L, of from 100 to 1000 L, or above 1000 L. The cultivating conditions may be controlled (e.g., pH, gas, temperature, etc.). An advantage of using bioreactors over traditional 2D monolayer cultures is its ability to increase cell density.

The cells may be cultivated e.g. as suspended cells, as adherent cells or as semi-adherent cells, on microcarriers (such as those disclosed in as described in Nienow et al., (Biochemical Engineering Journal, 2014, 85:79-88)), and on scaffolds or a combination thereof. Optionally, in the case of adherent cells (and related cultures, such as on scaffolds), the surface may be treated by plasma treatment, collagen coating, etc., to improve initial attachment of cell to either reach proliferate state (dividing and increase in size) or terminally differentiated state or rest-phase state to achieve cellular monolayer for subsequent growth steps (attached on the surface).

The initial density of cells in the nutrient composition (seeding density) is not particularly limited. For example, the seeding density may be in the range of from 1 × 10⁴ to 1 × 10⁷ cells/mL, preferably from 1 × 10⁵ to 1 × 10⁶ cells/mL, more preferably from 1.5 × 10⁵ to 3 × 10⁵ cells/mL.

Preferably, the cells are cultivated as suspended cells. In this case, the cell culture is preferably agitated in step 2). The means for agitation are not particularly limited and may be any means known in the art that are suitable for cell cultures. For example, agitation may be performed by stirring (also known as spinning), shaking, rocking, inverting or combinations thereof. The agitation by such means may be performed at any rate suitable for the cultivation process, and will depend on factors such as the size of the reactor, the viscosity of the culture medium, and the type of cells being cultivated, and can be adjusted to these factors by those skilled in the art.

Incubation of the cells in step 3) may be carried out under any suitable conditions sufficient for the cultivation of the selected cells. Cultivation may be understood in its broadest sense and may e.g. include one or more processes of cell proliferation/multiplication, adaptation to serum-free media, adaptation to suspension cultivation, genetic engineering, (trans)-differentiation, protein expression and others. In a preferred embodiment, cultivating the cells involves cell proliferation and preferably is conducted until the cell count of said cells has multiplied by at least 2-fold or more, 3-fold or more, 4-fold or more, 5-fold or more, 10-fold or more, or 25-fold or more.

Preferably, the cells of interest are cultured under sterile and controlled conditions to prevent contamination and provide beneficial growth conditions. External factors such as temperature, pH and humidity may be controlled. Cultivation may be performed at laboratory-grade incubators or in industrial large scale. For mammalian cells, cells growth conditions may, for example be: 34 to 39 °C (e.g. 37°C), approximately 5% by weight CO₂, approximately 95% air, and approximately 85-95% relative humidity. The culturing conditions may be adapted to the respective cells of interest and the nutrient composition, e.g. the buffer system employed therein. For avian cells, culturing temperature may be, e.g. 38 to 42°C. For example, at laboratory scale, cells may be cultivated in glass or plastic petri dishes, glass flasks, or plastic wears such as plastic flasks. Typically, a pH range is cellular physiological range (pH 7.0-7.4). Optionally, a cell culture device may be pre-coated (e.g. with a protein such as, e.g., collagen (type I), gelatin), which may improve initial attachment of the cells.

During cell cultivation, the nutrient composition of the invention in the cell culture may be exchanged and/or supplemented by additional nutrient composition or individual components thereof. This may be necessary depending on the consumption of components and/or the generation of undesired metabolites or other contaminants in the cell culture.

Another aspect of the present invention is a cellular material obtained by the method for the cultivation described above. The cellular material will be typically obtained by harvesting the cells cultivated in the method described above. For example, harvesting of the cells to provide cell the cellular material may be conducted by any means known in the art. When the cells are cultivated as adherent cells or as semi-adherent cells, harvesting the cells may comprise detaching the cells from the substrate. This may be performed mechanically (e.g., by scratching) or supported by one or more enzymes (e.g., trypsin) as generally known in the art, or by adding certain salts (e.g. citrate salts, such as sodium citrate, optionally in a buffer solution, such as phosphate buffered saline). A cell suspension may be obtained.

In one embodiment, the harvesting of the cells further comprises at least partly separating the cells from the cell culture medium. This may be achieved by any means. In a preferred embodiment, harvesting includes the removal of cell culture medium. In such step, at least 10% by weight, at least 25% by weight, at least 50% by weight, at least 70% by weight, at least 80% by weight, at least 90% by weight, at least 95% by weight, at least 98% by weight, or at least 99% by weight, referred to the total amount of cell culture medium present at the end of cultivating step 3), or (essentially) all cell culture medium present at the end of cultivating step 3), may be removed.

The at least partly removal of the cell culture medium from the cells may be achieved by any means. In a preferred embodiment, the harvesting includes the removal of cell culture medium by means of centrifugation, filtration, dialysis, or rinsing adherent cells.

Such step may optionally include optional washing steps with one or more buffers. Such washing step may also remove remaining and metabolites. In a preferred embodiment, the harvesting includes the removal of cell culture medium by means of centrifugation and optional washing steps with one or more buffers.

Optionally, the removed cell culture medium or water thereof may be recycled. Optionally, it may be used in the process as cell culture medium in the cultivation method of the invention. A waste management may be used to further use the nutrients contained in the consumed cell culture medium. Thus, optionally, also the not yet consumed nutrients may be recycled to step the method.

In an alternative embodiment, the harvesting includes the maintenance of at least parts of cell culture medium and a step of pasteurization.

Optionally, the cells may be resolubilized, e.g. in a buffered solution. Optionally, such buffered solution may contain a buffer agent selected from the group consisting of histidine, acetate, citrate, glycine, phosphate, or tris(hydroxymethyl)-aminomethane (TRIS).

Optionally, harvesting of the cells comprises a further step of rendering at least a part of the cells inviable, in particular breakup of the cells. This may be achieved by any means, such as, e.g., freeze-thaw cycles, lyophilization, applying ultrasound, mechanical stress (e.g, in a French press or a homogenizer), or exposure to detergents (such as Octoxinol 9, Triton X-100 and NP-40). This may provide a cellular material that contains dead cell-derived material. Such dead cell-derived material may comprise cell membranes and fractions thereof and inner parts of the cells such as cell lysate, including cytoplasm and/or cellular organelles.

In an alternative embodiment, viable cells may be harvested. This may optionally be achieved by cultivating cells in a suspension culture. Then, the cells including the cell culture medium may be further used as a cellular material. In an alternative embodiment, viable cells may be harvested e.g. by applying moderate centrifugal forces or filtration at low shear stress to cells either cultivated in a suspension culture or attached cells previously detached by mild means such as enzymes. Cells may also be harvested as described in Nienow et al., (Biochemical Engineering Journal, 2014, 85:79-88).

In one embodiment, the cells or fragments thereof are separated from the cell culture medium and represent the cellular material without the cell culture medium. In another embodiment, the cellular material still contains at least parts of the cell culture medium.

Optionally, the cellular material may be preserved, e.g. to prevent spoilage. The step of preserving the cellular material from spoiling may be conducted by any means. For example, the cellular material may be lyophilized, pasteurized, fermented, pickled, smoked, or dried to avoid microbial growth. Alternatively or additionally, the product may be heated, while reducing sugars such as glucose, lactose, and maltose may optionally be added to achieve a Maillard reaction during heating. Alternatively or additionally, preserving agents (e.g., benzoic acid) may optionally be added.

The cellular material of the invention may be used, e.g. as a source of cells for further cultivation processes, provided that the cells are still viable, or may be used e.g. in the production of food such as cultivated meat, or food supplements.

The invention is further illustrated by the examples, figures and claims.

### EXAMPLES

For the nutrient compositions described below, following components were used.
- E6:: Gibco^{™} Essential 6^{™} serum-free culture medium was acquired from ThermoFisher Scientific; E6 contained L-ascorbic acid-2-phosphate magnesium salt as an antioxidant, sodium selenite, insulin and transferrin, in DMEM/F12 as a basal nutrient medium;
- CHO:: Culture medium optimized for suspension cultivation of Chinese Hamster Ovary cells ("CHO medium serumfrei"), was acquired from Bio&Sell; CHO was a basal nutrient medium containing yeast extract;
- B27:: Gibco^{™} B-27^{™} serum free supplement (50X concentration) was acquired from ThermoFisher Scientific; B27 contained bovine serum albumin (BSA), antioxidants (catalase, glutathione, superoxide dismutase, tocopherol, tocopherol acetate), sodium selenite, insulin and transferrin;
- DMEM/F12:: Dulbecco's Modified Eagle Medium/Nutrient Mixture F-12 Ham (DMEM/F12, 1:1 Mixture; with 15 mM HEPES buffer) was acquired from HiMedia^{®} Laboratories. DMEM/F12 was used as a basal nutrient medium;
- TGFβ1:: HEK293-derived TGFβ1 was acquired from PeproTech^{®},
- FGF2:: FGF2-G3 was acquired from Defined Bioscience;
- Albumin: recombinant albumin was acquired from Sigma Aldrich.
- TrypLE: Gibco^{™} TrypLE^{™} Select recombinant enzyme was acquired from ThermoFisher Scientific;
- F-68: Pluronic^{®} F-68 surfactant was acquired from ThermoFisher Scientific;
- Panexin CD: Serum Replacement "Panexin CD" was acquired from PAN^{™} BIOTECH, containing recombinant proteins, lipids, salts, amino acids, trace elements and hormones;
- PAM: Preadipocyte Growth Medium was acquired from PromoCell;
- PBS: Phosphate-buffered saline (without Ca, Mg) was acquired from VWR.

Incubation of cell cultures was carried out in a CO₂ incubator (PHC MCO-230AICUV) at 37 °C, 5% CO₂.

Cell density measurements were carried out using a Vi-CELL BLU Cell Viability Analyzer (Beckman Coulter) or, where specified, with LUNA-FL^{™} Dual Fluorescence Cell Counter (Logos Biosystems) in adherent cultures, after dyeing with Acridine Orange/Propidium Iodide Stain (Logos Biosystems).

Images of the cell cultures were taken using a microscope (Leica DMIL LED FLUO) with camera attachment (Leica Flexacam C1).

Cell cultures were agitated either using 125 mL spinner flasks (Corning) and a magnetic stirrer (bioMIXdrive 4; 2mag AG), or using 125 mL shaker flasks (VWR) on an orbital shaker (ThermoFisher Scientific) or six well plates (Nunclon Sphere) on an orbital shaker (VWR).

Multiple nutrient compositions were prepared by mixing the components disclosed below. The compositions can be prepared by mixing the components other than the basal nutrient medium or concentrates thereof in basal nutrient medium in amounts required to obtain the required concentration in the final composition, and diluting the components with the basal nutrient medium. Typically, the volume of the components presented in µg/L or mg/L are negligible in the total volume.

### Nutrient composition Ex.1:

50 mL of E6,
49 mL of DMEM/F12,
1 mL of B27,
50 µg/L of FGF2,
1 µg/L of TGFβ1.

In an alternative approach, this composition was prepared by mixing the following components:
99 mL of DMEM/F12;
1 mL of B27;
9.7 mg/L of insulin;
5.35 mg/L of transferrin;
7 µg/L of sodium selenite;
32 mg/L of L-ascorbic acid-2-phosphate magnesium salt;
50 µg/L of FGF2;
1 µg/L of TGFβ1.

The resultant nutrient composition Ex.1 contained approximately 12 mg/L of insulin, 8 mg/L of transferrin, 13 µg/L of sodium selenite, 100 mg/L of antioxidants, 50 µg/L of FGF2, 1 µg/L of TGFβ1, and 800 mg/L of albumin.

As further components stemming from B27, Ex.1 also contained approximately 10 µg/L corticosterone, 3.15 µg/L of progesterone, 50 µg/L retinol acetate, 0.5 mg/L of linolenic acid, 0.5 mg/L of linoleic acid 0.5 mg/L of oleic acid, 0.5 mg/L of pipecolic acid, 1 µg/L of triiodothyronine; 0.5 µg/L, of ethanolamine; 8 mg/L, of putrescine; 7.5 mg/L, of galactose; 1 mg/L, of L-carnitine; and 1.25 mg/L of biotin. The antioxidants contained approximately 32 mg/L of L-ascorbic acid-2-phosphate magnesium salt stemming from E6, and 1.25 mg/L of catalase, 62.5 mg/L of glutathione, 1.25 mg/L of superoxide dismutase, 0.5 mg/L of tocopherol and 0.5 mg/L of tocopherol acetate, each stemming from B27.

### Nutrient composition C.1:

100 mL of CHO,
30 µg/L of FGF2.

The resultant nutrient composition C.1 contained 30 µg/L of FGF2 and further contained yeast extract.

### Nutrient composition Ex.2:

25 mL of nutrient composition Ex.1,
75 mL of nutrient composition C.1.

In an alternative approach, this composition was prepared by mixing the following components:
75 mL of CHO,
12.5 mL of E6,
12.25 mL of DMEM/F12,
0.25 mL of B27,
35 µg/L of FGF2,
0.25 µg/L of TGFβ1.

The resultant nutrient composition Ex.2 contained approximately 3 mg/L of insulin, 2 mg/L of transferrin, 3.3 µg/L of sodium selenite, 25 mg/L of antioxidants, 35 µg/L of FGF2, 0.25 µg/L of TGFβ1, and 200 mg/L of albumin, and further contained yeast extract.

### Nutrient composition Ex.3:

Nutrient composition Ex.1 was supplemented with the surfactant F-68 to a concentration of 0.1 vol.-%.

The resultant nutrient composition Ex.3 contained approximately 12 mg/L of insulin, 8 mg/L of transferrin, 13 µg/L of sodium selenite, 100 mg/L of antioxidants, 50 µg/L of FGF2, 1 µg/L of TGFβ1, and 800 mg/L of albumin, and further contained the surfactant.

### Nutrient composition Ex.4:

Nutrient composition Ex.1 was supplemented with ferric citrate to a concentration corresponding approximately to that in "CHO medium serumfrei".

The resultant nutrient composition Ex.4 contained approximately 12 mg/L of insulin, 8 mg/L of transferrin, 13 µg/L of sodium selenite, 100 mg/L of antioxidants, 50 µg/L of FGF2, 1 µg/L of TGFβ1, and 800 mg/L of albumin, and further contained the ferric citrate.

The above compositions were used for cell cultures in suspension as described below.

### Nutrient composition Ex.5:

50 mL of nutrient composition Ex.1,
50 mL of nutrient composition C.1

The resultant nutrient composition Ex.5 contained approximately 6 mg/L of insulin, 4 mg/L of transferrin, 6.6 µg/L of sodium selenite, 50 mg/L of antioxidants, 40 µg/L of FGF2, 0.5 µg/L of TGFβ1, and 400 mg/L of albumin, and further contained yeast extract.

### Nutrient composition C.2:

75 mL of CHO,
25 mL of E6,
35 µg/L of FGF2,
0.25 µg/L of TGFβ1.

The resultant nutrient composition C.2 contained approximately 5 mg/L of insulin, 3 mg/L of transferrin, 3.5 µg/L of sodium selenite, 16 mg/L of antioxidants, 35 µg/L of FGF2 and 0.25 µg/L of TGFβ1, and further contained yeast extract.

### Nutrient composition Ex.6:

75 mL of CHO,
25 mL of E6,
35 µg/L of FGF2,
0.25 µg/L of TGFβ1,
200 mg/L of albumin.

The resultant nutrient composition Ex.6 contained approximately 5 mg/L of insulin, 3 mg/L of transferrin, 3.5 µg/L of sodium selenite, 16 mg/L of antioxidants, 35 µg/L of FGF2, 0.25 µg/L of TGFβ1, and 200 mg/L of albumin and further contained yeast extract.

### Nutrient composition Ex.7:

75 mL of CHO,
24.5 mL of DMEM/F12,
0.5 mL of B27,
35 µg/L of FGF2,
0.25 µg/L of TGFβ1.

The resultant nutrient composition Ex.7 contained approximately 0.8 mg/L of insulin, 1.3 mg/L of transferrin, 3.1 µg/L of sodium selenite, 33 mg/L of antioxidants, 35 µg/L of FGF2, 0.25 µg/L of TGFβ1, and 400 mg/L of albumin, and further contained yeast extract.

### Nutrient composition C.3:

75 mL of CHO,
25 mL of DMEM/F12.

The resultant nutrient composition C.3 was substantially composed of basal nutrient medium and yeast extract.

### Nutrient composition C.4:

75 mL of CHO,
25 mL of DMEM/F12,
35 µg/L of FGF2,
0.25 µg/L of TGFβ1.

The resultant nutrient composition C.4 was substantially composed of basal nutrient medium, growth factors and yeast extract.

### Nutrient composition C.5:

75 mL of CHO,
24.5 mL of DMEM/F12,
0.5 mL of B27.

The resultant nutrient composition C.5 contained approximately 0.8 mg/L of insulin, 1.3 mg/L of transferrin, 3.1 µg/L of sodium selenite, 33 mg/L of antioxidants and 400 mg/L of albumin, and further contained yeast extract.

### Nutrient composition C.6:

Nutrient composition C.6 was prepared as described in Kolkmann et al. (Frontiers in Bioengineering and Biotechnology 2022, 10, Article 895289, 1-15).

### Nutrient composition C.7:

Nutrient composition C.7 was prepared as described in Tan et al. (Cytotherapy 2015, 17, 1152-1165) and Domnina et al. (Journal of Personalized Medicine 2021, 11, 466), however, without FGF2 and TGFβ1.

### Nutrient composition C.8:

97.5 mL of DMEM/F12;
2.5 mL of PanexinCD
9.7 mg/L of insulin;
5.35 mg/L of transferrin;
7 µg/L of sodium selenite;
32 mg/L of L-ascorbic acid-2-phosphate magnesium salt;
50 µg/L of FGF2;
1 µg/L of TGFβ1;
800 mg/L of albumin.

### Nutrient composition C.9:

100 mL of DMEM/F12
19.4 mg/L of insulin;
10.7 mg/L of transferrin;
14 µg/L of sodium selenite;
64 mg/L of L-ascorbic acid-2-phosphate magnesium salt;
100 µg/L of FGF2;
2 µg/L of TGFβ1;
800 mg/L of albumin.

### Nutrient composition C.10:

100 mL of DMEM/F12
19.4 mg/L of insulin;
10.7 mg/L of transferrin;
14 µg/L of sodium selenite;
64 mg/L of L-ascorbic acid-2-phosphate magnesium salt;
100 µg/L of FGF2;
2 µg/L of TGFβ1;
2000 mg/L of albumin.

The above compositions were used for cultivation of porcine muscle-derived cells in suspension, or for cultivation of bovine fat-derived cells in adherent culture, as described below.

### Suspension Cultures

### Example 1 (nutrient composition Ex.1)

2.5 mL of a suspension containing a porcine muscle-derived cell line adapted to suspension cultivation in nutrient composition Ex.1 were transferred to a 125 mL shaker flask, and 17.5 mL of fresh Ex.1 were added, to obtain a culture with a viable cell density of 0.3 × 10⁶ cells/mL. The flask was placed into an incubator and shaken at 100 rpm.

After 10 days, a 300 µL sample was taken for analysis, and 20 mL of Ex.1 were added to the shaker flask. The flask was placed into the incubator and shaken at 100 rpm.

After 2 more days, the shaking speed was increased to 120 rpm and fresh FGF2 was added (50 µg/L).

After 4 more days, a another 300 µL sample was taken for analysis.

The remaining cells were centrifuged and treated with TrypLE at 37 °C for 3 min. The cells were resuspended in 15 mL of fresh Ex.1 and 5 mL of conditioned Ex.1.

Final analysis was performed after 28 more days.

Cell proliferation and viability were observed throughout the cultivation.

At the first analysis, few small and medium-sized cell aggregates were observed At the final analysis, viability of the cells was determined to be between 70 and 80%.

### Comparative Example 1 (nutrient composition C.1)

Example 1 was repeated, however the nutrient composition Ex.1 was replaced by nutrient composition C.1, obtained by adding 30 µg/L of FGF2 to CHO.

At the first analysis, cell proliferation and viability were observed, but also, many large cell aggregates were observed.

At the second analysis no cell proliferation was observed, and no substantial cell viability was observed.

### Example 1.1 and Example 2 (nutrient compositions Ex.1 and Ex.2)

Cell cultures with a viable cell density of 1.5 × 10⁵ cells/mL were prepared in accordance with Example 1 using nutrient composition Ex.1 and nutrient composition Ex.2, and were incubated for 168 hours in shaker flasks, at a shaking speed of 125 rpm. The cumulative cell count was determined after 72 hours, 90 hours, 96 hours and 168 hours, using a Vi-CELL BLU Cell Viability Analyzer (Beckman Coulter). Aggregate formation was analysed under a microscope after 2, 4 and 7 days.

In Example 1.1, a cumulative cell count of about 4 × 10⁵ cells/mL was determined after 168 hours, and cell viability was between 70 and 80%. Cell proliferation was observed throughout the cultivation. The microscopy images showed a mixture of single cells in suspension and multiple medium-sized and large cell aggregates.

In Example 2, the cumulative cell count was about 15 × 10⁵ cells/mL after 168 hours, and cell viability was above > 95%. Cell proliferation was observed throughout the cultivation. The microscopy images showed a mixture of mostly single cells in suspension with few small cell aggregates.

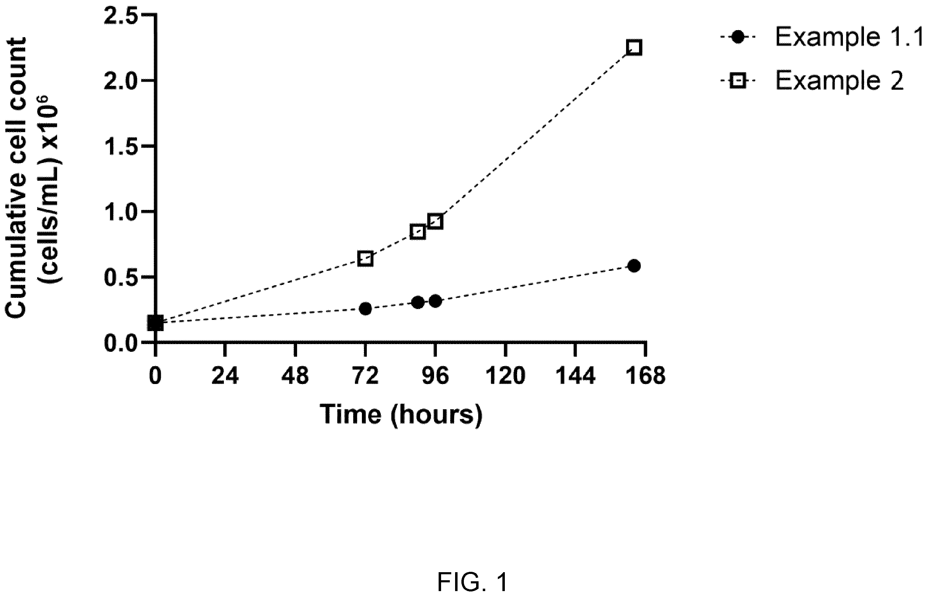

**FIG. 1** shows the development of the cumulative cell count for Example 1.1 and Example 2.

**FIG. 2A** shows the microscopy images of Example 1.1 after 2, 4 and 7 days.

**FIG. 2B** shows the microscopy images of Example 2 after 2, 4 and 7 days.

From the comparison of Example 1.1 and Example 2 it is evident that, while the composition of the invention without yeast extract (Ex.1) already performs well in suspension cultures, the composition of the invention comprising yeast extract (Ex.2) performs substantially better in terms of cell viability and cell proliferation, and substantially reduces aggregate formation, although the overall concentration of major components such as of insulin, transferrin, sodium selenite, antioxidants, growth factors and albumin is reduced.

### Examples 3 and Example 4 (nutrient compositions Ex.3 and Ex.4)

In order to investigate whether the improvements observed in Example 2 compared with Example 1.1 can be attributed to other components of "CHO Medium serumfrei" than yeast extract, Example 1.1 was repeated, however composition Ex.1 was replaced with composition Ex.3 comprising Pluronic^{®} F-68, or composition Ex.4 comprising ferric citrate.

All other components known for "CHO Medium serumfrei" (salts, amino acids, vitamins, trace elements, L-glutamine, HEPES buffer), with the exception of the ultra-filtered yeast extract, Pluronic^{®} F-68 and ferric citrate, were already present in nutrient composition Ex.1, and thus were deemed not responsible for the observed improvement.

In Example 3, cumulative cell count, cell viability and cell proliferation were essentially the same as in Example 1.1, however, the number and size of cell aggregates was substantially decreased.

In Example 4, the results were essentially the same as in Example 1.1.

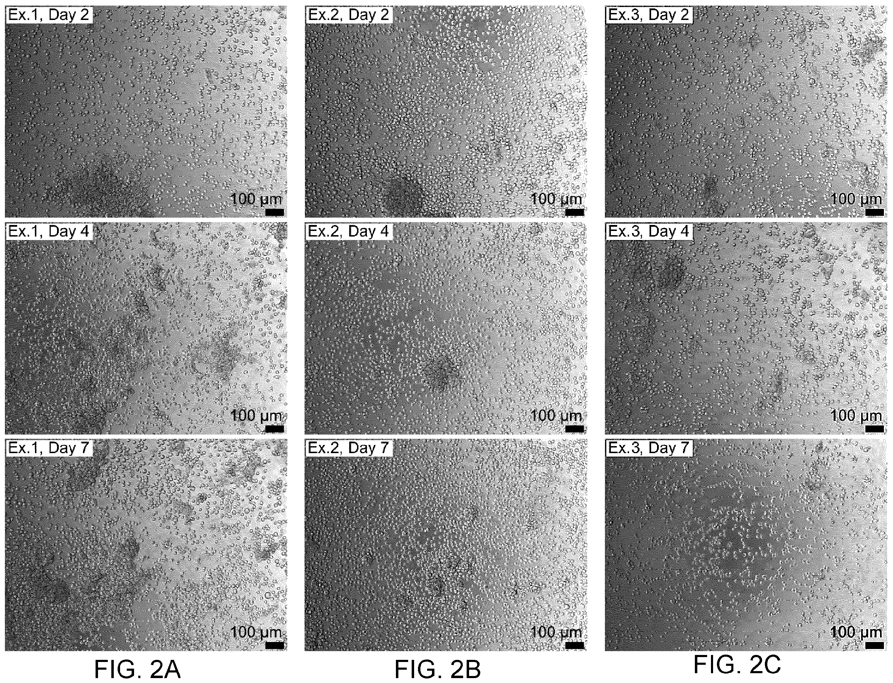

**FIG. 2C** shows the microscopy images of Example 3 after 2, 4 and 7 days.

### Example 2.1 and Example 5 (nutrient compositions Ex.2 and Ex.5)

From a cell culture in Ex.2, obtained as in Example 2, cells were harvested and single cells were enriched using a 70 µm cell strainer. Cells were then centrifuged at 300 g for 5 min, the supernatant aspirated and the pellets resuspended in 10 mL of the nutrient composition Ex.2 or the nutrient composition Ex.5 for cell count measurement. Two spinner flasks were inoculated with the cells at a seeding density of 0.15 ×10⁶ cells/mL with an initial total volume of 40 mL for each spinner flask.

Cells were cultivated in single use spinner flasks (125 mL) with a stirring rate of 100 rpm throughout the whole cultivation process. Each day a sample was taken from the cultures for analysis, and after 70 h, 32 mL of respective medium was added to the respective culture.

Cell proliferation and viability were observed throughout the cultivation in both nutrient compositions Ex.2 and Ex.5. In the final analysis after 144 h, viability of the cells was determined to be > 90% both in Ex.2 and Ex.5, and the viable cell density was about 0.4 × 10⁵ cells in both nutrient compositions. Few small cell aggregates were formed throughout the cultivation.

From Examples 2.1 and 5, it becomes evident that the amount of individual components in the nutrient compositions can be varied in a broad range, without substantially affecting cell viability, cell proliferation and cell aggregate formation at the tested cell densities. Moreover, these examples show that agitation by stirring is a viable alternative to agitation by shaking.

### Comparative Example 2 (nutrient composition C.2)

From a cell culture in Ex.2, obtained as in Example 2, cells were harvested and single cells were enriched using a 70 µm cell strainer.

Cells were then centrifuged at 300 g for 5 min, the supernatant aspirated and the pellets were re-suspended in 10 mL of the nutrient composition C.2, to obtain a culture with a viable cell density of 0.3 × 10⁶ cells/mL.

The cells were allowed to adapt to the nutrient composition C.2 for 2 weeks and further were allowed to adapt to the nutrient composition Ex.6 (described below) for 1 week) while being agitated by a shaker.

The adapted cell culture having a viable cell density of 0.3 × 10⁶ cells/mL was placed into an incubator and shaken at 125 rpm. After 72 h of incubation, a sample (300 µL) was taken for analysis, and the culture medium was exchanged by the same amount of fresh C.2.

Further samples were taken after a total of 96 h, 120 h and 144 h of incubation. After a total of 144 h of incubation, the cell culture was diluted by adding 10 mL of fresh C.2, and the cell culture was further incubated up to a total of 168 h, after which another sample was taken for analysis.

Cell proliferation and viability were observed throughout the cultivation.

In the final analysis, viability of the cells was determined to be > 90%. However, large cell aggregates were observed.

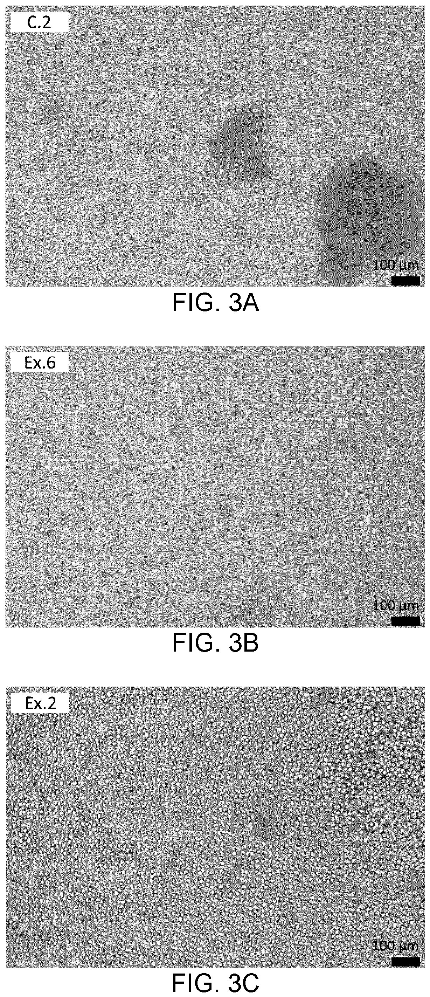

**FIG. 3A** shows a microscopy image of the cell culture in the nutrient composition C.2 at final analysis.

### Example 6 (nutrient composition Ex.6)

After adaptation to the nutrient compositions C.2 and Ex.6 as disclosed in Comparative Example 2, the subsequent steps were carried out in the same manner as in Comparative Example 2, however, nutrient composition C.2 was replaced with nutrient composition Ex.6.

Cell proliferation and viability were observed throughout the cultivation.

At the final analysis, viability of the cells was determined to be > 90%, and only few small cell aggregates were observed.

**FIG. 3B** shows a microscopy image of the cell culture in the nutrient composition Ex.6 at final analysis.

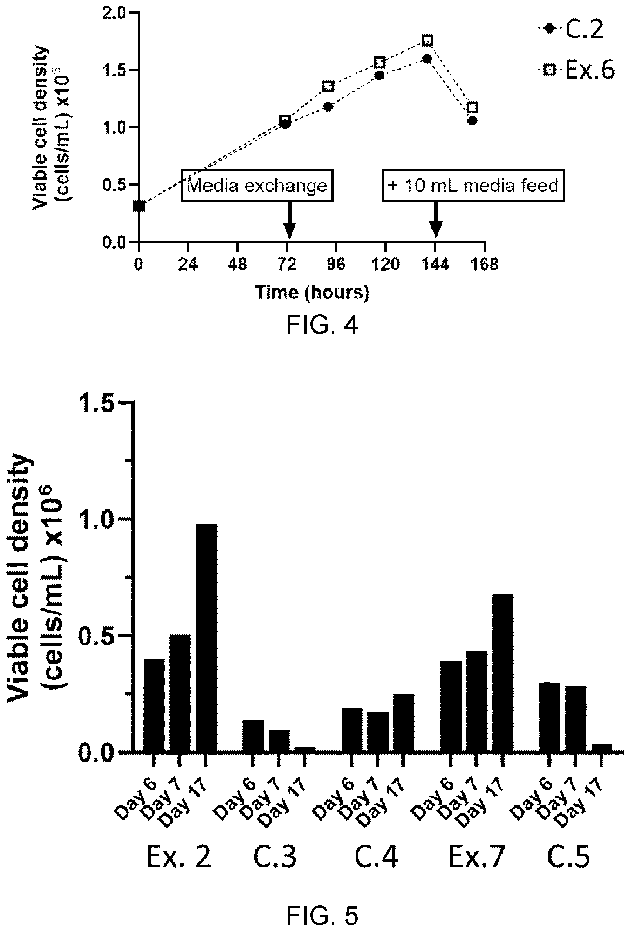

**FIG. 4** shows a comparison of the increase in viable cell density of the cell cultures in nutrient compositions C.2 and Ex.6.

### Example 2.2 (nutrient composition Ex.2)

For further comparison with Comparative Example 2 and Example 6, a cell culture in nutrient composition Ex.2 was analyzed under a microscope at similar lighting conditions and the same magnification.

**FIG. 3C** shows a microscopy image of the cell culture in the nutrient composition Ex.2.

Comparison of Example 6 and Example 2.2 shows that recombinant albumin (Example 6) can be used as a substitute for the supplement B-27 containing bovine serum albumin (Example 2.2). However, as can be seen from Comparative Example 2, aggregate formation is substantially increased when albumin is absent, even if the composition comprises a surfactant (as part of "CHO Medium serumfrei"), which was shown to reduce aggregate formation in the presence of albumin (see Example 3).

### Examples 2.3 and 7 (nutrient compositions Ex.2 and Ex.7) and Comparative Examples 3, 4 and 5 (nutrient compositions C.3, C.4 and C.5)

From a cell culture in Ex.2, obtained as in Example 2, cells were harvested and single cells were enriched using a 70 µm cell strainer. Cells were then centrifuged at 300 g for 5 min, the supernatant aspirated and the pellets were re-suspended in 2 mL of the respective nutrient composition, each seeded in one well of a six well plate, to obtain cultures with a viable cell density of 0.3 × 10⁶ cells/mL. The six well plate was agitated on an orbital shaker at a shaking speed of 90 rpm.

On day 6, cells were detached using TrypLE and cell suspension was pooled with the respective supernatant. The cells were then centrifuged and the resulting cell pellet re-suspended in 4 mL of the respective medium.

The obtained cell suspension was seeded in a fresh cell-repellent six well plate, and agitated on the orbital shaker at a shaking speed of 90 rpm.

The viable cell density for each culture was determined on the same day (day 6) and on days 7 and 17.

Cell proliferation and viability were observed throughout the cultivation in nutrient composition Ex.2 and nutrient composition Ex.7. In nutrient composition Ex. 2, the viable cell density was determined to be about 0.4 × 10⁶ cells/mL on day 6, increasing to about 1 × 10⁶ cells/mL on day 17. In nutrient composition Ex.7, the viable cell density increased from about 0.4 × 10⁶ cells/mL on day 6 to about 0.7 × 10⁶ cells/mL on day 17.

While both Ex.2 and Ex.7 achieve desirable results, it can be seen from this comparison that nutrient compositions of the invention comprising yeast extract perform better in terms of cell proliferation, if prepared from a pre-formed nutrient composition according to the invention lacking yeast extract (e.g. using Ex.1, as in Example 2.3).

Without being bound by theory, it is believed that the ratio of individual components present in or similar to Ex.1 (as opposed to a mixture of basal nutrient media, B27 and growth factors), is useful for achieving such improvement.

In nutrient composition C.3, a viable cell density of about 0.14 × 10⁶ cells/mL was determined on day 6, but decreased substantially to <0.1 cells/mL on day 17. In nutrient composition C.4, a viable cell density of about 0.2 × 10⁶ cells/mL was determined on day 6, which slightly increased to about 0.25 × 10⁶ cells/mL on day 17.

In nutrient composition C.5, a viable cell density of about 0.3 × 10⁶ cells/mL was determined on day 6, which decreased substantially to <0.1 cells/mL on day 17.

**FIG. 5** shows a comparison of the viable cell densities for cell cultures in nutrient compositions Ex.2, C.3, C.4, Ex.7 and C.5 (from left to right) at days 6, 7 and 17.

The approximate amounts of components in the nutrient compositions, which are not considered to be part of a basal nutrient mixture, are summarized in the Table 2 below.

Moreover, results for cell proliferation and cell viability of porcine muscle-derived cells in suspension were evaluated according to the following criteria:
Cell proliferation:
   - +: viable cell density increased throughout incubation;
   - /: viable cell density stagnant between final two analyses;
   - -: viable cell density declined between final two analyses.
Cell viability:
   - +: cell viability at least 90% at final analysis;
   - /: cell viability at least 70% and below 90% at final analysis;
   - -: cell viability below 70% at final analysis.

Aggregate formation was evaluated by comparison with other nutrient compositions used in cultures under substantially the same conditions.

The table indicates for each example, which other nutrient compositions performed worse than the nutrient composition of the respective column (i.e. which compositions had larger and more aggregates), or which other nutrient compositions performed similarly to the composition of the respective column. "n.d." means that no evaluation of aggregate formation was carried out. "-" means that the nutrient composition performed worst in all comparisons.

**Table 2**

| | Ex.1/Ex.4 | C.1 | Ex.2 | Ex.3 | Ex.5 | C.2 | Ex.6 | Ex.7 | C.3 | C.4 | C.5 |
|---|---|---|---|---|---|---|---|---|---|---|---|
| Insulin [mg/L] | 12 | 0 | 3 | 12 | 6 | 5 | 5 | 0.8 | 0 | 0 | 0.8 |
| Transferrin [mg/L] | 8 | 0 | 2 | 8 | 4 | 3 | 3 | 1.3 | 0 | 0 | 1.3 |
| Sodium Selenite [µg/L] | 13 | 0 | 3.3 | 13 | 6.6 | 3.5 | 3.5 | 3.1 | 0 | 0 | 3.1 |
| Antioxidants [mg/L] | 100 | 0 | 25 | 100 | 50 | 16 | 16 | 33 | 0 | 0 | 33 |
| Albumin [mg/L] | 800 | 0 | 200 | 800 | 400 | 0 | 200 | 400 | 0 | 0 | 400 |
| FGF2 [µg/L] | 50 | 30 | 35 | 50 | 40 | 35 | 35 | 35 | 0 | 35 | 0 |
| TGFβ1 [µg/L] | 1 | 0 | 0.25 | 1 | 0.5 | 0.25 | 0.25 | 0.25 | 0 | 0.25 | 0 |
| Yeast extract present? | No | Yes | Yes | No | Yes | Yes | Yes | Yes | Yes | Yes | Yes |
| Surfactant present? | No | Yes | Yes | Yes | Yes | Yes | Yes | Yes | Yes | Yes | Yes |
| Cell proliferation | + | - | + | + | + | + | + | + | - | / | - |
| Cell viability | / | - | + | / | + | + | + | + | - | / | - |
| Less/smaller aggregates than in: | C.1 | | Ex.1 | Ex.1 | | | | n.d. | n.d. | n.d. | n.d. |
| | | - | Ex.3 | Ex.4 | | - | | | | | |
| Aggregate number/size similar to: | | | Ex.5 | | Ex.2 | | Ex.2 | n.d. | n.d. | n.d. | n.d. |
| | | - | Ex.6 | | | - | | | | | |

### Adherent Cultures

### Example 1.2 (nutrient composition Ex.1) and

### Comparative Examples 6 to 10 (nutrient compositions C.6 to C.10)

Cells from an adherent culture of a bovine fat-derived cell line were washed with PBS, detached using TrypLE, and placed into a flask, after which trypsin was deactivated by addition of PAM. Cells were counted in suspension using the LUNA-FL^{™} Counter.

Cells in a mixture of 2 mL PAM containing 30 µg/L of FGF2 were seeded onto each well of a collagen-coated 6 well plate at 0.6×10⁵ cells per well, and incubated for 24 h. After this, the cells were washed twice with PBS and the media were exchanged in each well by one of the nutrient compositions Ex.1, C.6, C.7, C.8, C.9 and C.10.

The resultant cell cultures were then incubated for 3 days, followed by a media change using fresh media, and further incubation for 4 days.

After this incubation time, most cells in nutrient compositions C.6, C.7, C.8, C-9 and C.10 were dead and detached from the surface. In nutrient composition Ex.1, proliferation continued for at least 15 more days (including further media exchanges), at which point the experiment was stopped.

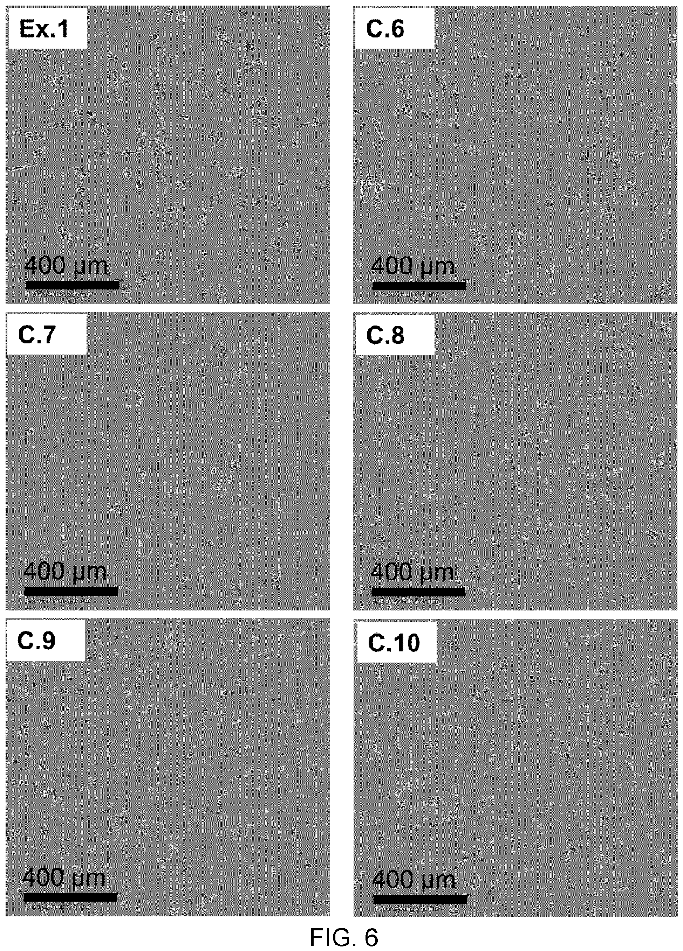

**FIG. 6** shows microscope images taken after 4 days of total incubation. The figure shows highest performance for Ex.1, where the fraction of cells that are viable and attached to the surface (visible as elongated, irregular structures) was highest among all cultures. In C.6 and C.10, a small fraction of viable cells was still observed. In all other media, most cells were detached from the surface and showed morpholgical signs of cell death (visible as essentially round spots and cell fragments).

The results demonstrate that adherent cultures of bovine fat-derived cells proliferate best in the nutrient composition Ex.1, which contains the additional components from B27.

### Example 1.3 (nutrient composition Ex.1) and

### Comparative Example 6.1 (nutrient composition C.6)

Cells from an adherent culture of a bovine fat-derived cell line were washed with PBS, detached using TrypLE, and re-suspended in DMEM/F12.

The cell suspension was centrifuged to remove excess Trypsin, and the supernatant was discarded without disturbing the cell pellet. The cell pellet was then re-suspended in DMEM-F12, and the cells were counted in suspension using the LUNA-FL^{™} Counter.

Cells in 4 mL of each of nutrient compositions Ex.1 and C.6 were seeded into cell culture flasks at 2.5×10⁵ cells per flask, and 12.5 µL of laminin (0.5 g/L solution) were added to each flask, to reach 0.25 µg/cm².

The cultures were incubated for 22 days, during which time passaging was carried out at least 4 times. When passaging cells, the cumulative cell count and cell viability were determined.

In nutrient composition Ex.1, the cumulative cell count reached approximately 3.5×10⁶ cells on day 22, and the cells were still proliferating. Viability was determined to be between 86% and 93% throughout the cultivation process (93% on day 21).

In nutrient composition C.6, cumulative cell count reached approximately 1.2×10⁶ cells on day 14, and stagnated at this value. Viability dropped below 80% on day 18. On day 22, no viable cells were detected.

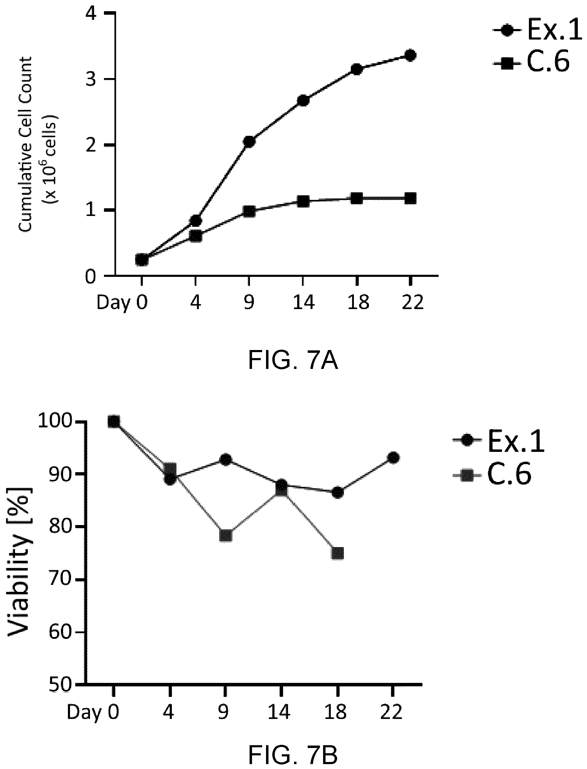

**FIG. 7A** shows the development of cumulative cell count throughout the cultivation processes in Ex.1 and C.6.

**FIG. 7B** shows the development of viability throughout the cultivation processes in Ex.1 and C.6.

### Example 1.4 (nutrient composition Ex.1) and

### Comparative Example 9.2 (nutrient composition C.9)

Cells from an adherent culture of a bovine fat-derived cell line were washed with PBS, detached using TrypLE, and re-suspended in DMEM/F12. The cell suspension was centrifuged to remove excess Trypsin, and the supernatant was discarded without disturbing the cell pellet.

The cell pellet was then re-suspended in DMEM-F12, and the cells were counted in suspension using the LUNA-FL^{™} Counter.

Cells in 2 mL of each of nutrient compositions Ex.1 and C.9 were seeded onto a 6-well plate at 0.6×10⁵ cells per well, and laminin was added to each well to reach 0.25 µg/cm².

The cultures were incubated for 4 days after which they were analyzed under a microscope.

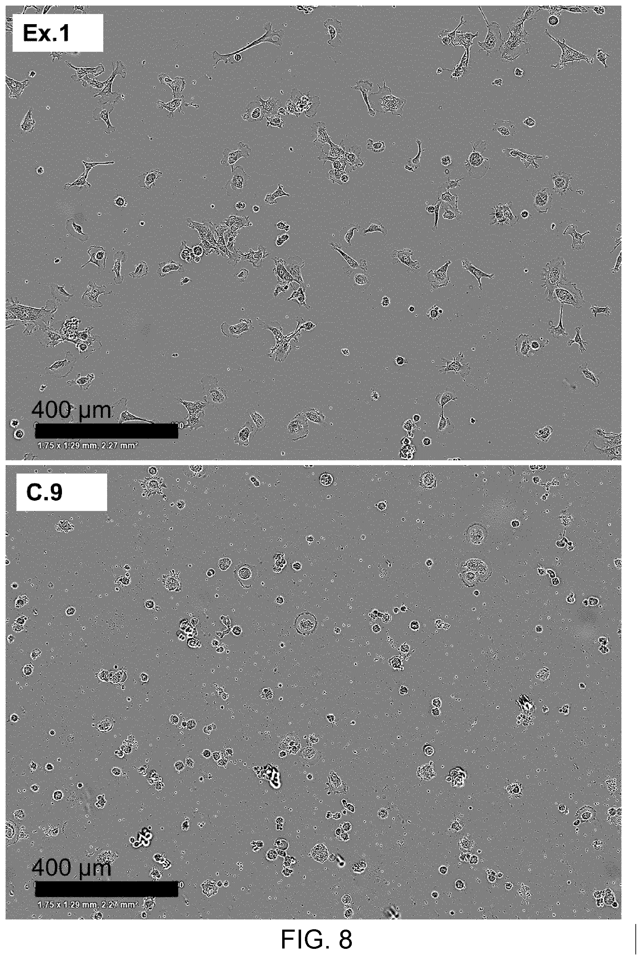

**FIG. 8** shows microscope images taken after 4 days of total incubation.

The figure shows highest performance for Ex.1, where most cells were viable and proliferated readily (visible as elongated, irregular structures).

In nutrient composition C.9, almost all cells were detached from the surface and showed morpholgical signs of cell death (visible as essentially round spots and cell fragments).

## Claims

1. A nutrient composition comprising the following components:
a) at least one basal nutrient medium;
b) at least one antioxidant, preferably ascorbic acid or a derivative thereof, more preferably L-ascorbic acid-2-phosphate or a salt thereof;
c) at least one source of selenium, preferably sodium selenite;
d) at least one insulin;
e) at least one transferrin; and
f) at least one growth factor;
g) at least one albumin; and
h) optionally at least one extract, protein isolate and/or hydrolysate of at least one plant or fungus, preferably yeast,
wherein the nutrient composition is free of animal-derived serum.

2. The nutrient composition according to claim 1, wherein the nutrient composition comprises the at least one extract, protein isolate and/or hydrolysate of at least one plant or fungus, preferably yeast extract.

3. The nutrient composition according to claim 1 or 2, wherein at least one basal nutrient medium comprises at least one culture medium for cell cultivation in suspension, preferably for cultivation of Chinese hamster ovary (CHO) cells in suspension.

4. The nutrient composition according to any one of claims 1 to 3, wherein the at least one albumin is plant albumin or recombinant albumin.

5. The nutrient composition according to any one of claims 1 to 4, wherein the at least one growth factor is a fibroblast growth factor receptor agonist, a transforming growth factor beta receptor agonist or a combination thereof, preferably modified or unmodified FGF2, modified or unmodified TGFβ1, or a combination thereof, more preferably a combination of FGF2 and TGFβ1.

6. The nutrient composition according to any one of claims 1 to 5, comprising, based on the total volume of the nutrient composition:
from 3 to 200 mg/L, preferably from 6 to 64 mg/L, more preferably from 8 to 40 mg/L, more preferably from 8 to 20 mg/L, of the at least one antioxidant,
preferably ascorbic acid or a derivative thereof, more preferably L-ascorbic acid-2-phosphate or a salt thereof, more preferably L-ascorbic acid-2-phosphate magnesium salt;
from 0.5 to 40 µg/L, preferably from 1 to 25 µg/L, more preferably from 1.5 to 15 µg/L, more preferably from 1.75 to 10 µg/L, of sodium selenite;
from 0.5 to 100 mg/L, preferably from 1.8 to 50 mg/L, more preferably from 2 to 20 mg/L, more preferably from 2.4 to 10 mg/L, of insulin;
from 0.5 to 20 mg/L, preferably from 1 to 15 mg/L, more preferably from 1.2 to 9 mg/L, more preferably from 1.3 to 5 mg/L, of transferrin;
from 4 to 100 µg/L, preferably from 8 to 80 µg/L, more preferably from 10 to 70 µg/L, more preferably from 30 to 40 µg/L, of a fibroblast growth factor receptor agonist, preferably modified or unmodified FGF2;
optionally from 0.1 to 4 µg/L, preferably from 0.2 to 3 µg/L, more preferably from 0.25 to 2 µg/L, more preferably from 0.25 to 1 µg/L, of a transforming growth factor beta receptor agonist, preferably modified or unmodified TGFβ1;
from 25 to 1000 mg/L, preferably 50 to 800 mg/L, more preferably 100 to 500 mg/L, more preferably 150 to 350 mg/L, of albumin; and
optionally from 10 to 5000 mg/L, preferably from 25 to 2000 mg/L, more preferably from 50 to 1500 mg/L, more preferably from 100 to 1000 mg/L, of yeast extract.

7. The nutrient composition according to any one of claims 1 to 6, wherein the basal nutrient medium comprises DMEM/F12, preferably wherein the ratio of DMEM:F12 is from 1:3 to 3:1, more preferably from 1:2 to 2:1, more preferably 1:1.

8. The nutrient composition according to any one of claims 1 to 7, comprising one or more, preferably all components selected from the group consisting of inorganic salts, amino acids and salts thereof, amines and salts thereof, vitamins, fatty acids, carbohydrates, nucleosides, enzymes, co-enzymes and hormones.

9. The nutrient composition according to any one of claims 1, 3 to 5, 7 or 8, wherein the nutrient composition does not contain an extract, protein isolate and/or hydrolysate of at least one plant or fungus, and comprises the at least one basal nutrient medium and, based on the total volume of the nutrient composition:
comprises, based on the total volume of the nutrient composition:
from 25 to 45 mg/L, preferably from 27 to 40 mg/L, more preferably from 30 to 35 mg/L, of ascorbic acid or a derivative thereof;
from 0.9 to 1.6 mg/L, preferably from 1 to 1.5 mg/L, more preferably from 1.2 to 1.3 mg/L, of at least one catalase;
from 45 to 80 mg/L, preferably from 50 to 75 mg/L, more preferably from 55 to 70 mg/L, of glutathion;
from 0.9 to 1.6 mg/L, preferably from 1 to 1.5 mg/L, more preferably from 1.2 to 1.3 mg/L, of at least one superoxide dismutase;
from 0.35 to 0.65 mg/L, preferably from 0.4 to 0.6 mg/L, more preferably from 0.45 to 0.55 mg/L, of tocopherol; and
from 0.35 to 0.65 mg/L, preferably from 0.4 to 0.6 mg/L, more preferably from 0.45 to 0.55 mg/L, of tocopherol acetate;
from 12 to 15 µg/L, preferably from 13 to 14 µg/L, more preferably from 13.2 to 13.5 µg/L of sodium selenite;
from 9 to 15 mg/L, preferably from 10 to 13.5 mg/L, more preferably from 10.5 to 12 mg/L of at least one insulin;
from 7.3 to 9.4 mg/L, preferably from 7.5 to 8.7 mg/L, more preferably from 7.6 to 8.2 mg/L of at least one transferrin;
from 30 to 70 µg/L, preferably from 40 to 60 µg/L, more preferably from 45 to 55 µg/L of FGF2;
from 0.5 to 1.5 µg/L, preferably from 0.8 to 1.2 µg/L, more preferably from 0.9 to 1.1 µg/L of TGFβ1;
from 500 to 1200 mg/L, preferably from 600 to 1000 mg/L, more preferably from 750 to 850 mg/L of at least one albumin;
from 5 to 15 µg/L, preferably from 7 to 13 µg/L, more preferably from 9 to 11 µg/L, of corticosterone;
from 2.2 to 4.1 µg/L, preferably from 2.5 to 3.8 µg/L, more preferably from 2.8 to 3.5 µg/L, of progesterone;
from 35 to 65 µg/L, preferably from 40 to 60 µg/L, more preferably from 45 to 55 µg/L, of retinol acetate;
from 0.35 to 0.65 mg/L, preferably from 0.5 to 0.6 mg/L, more preferably from 0.45 to 0.55 mg/L, of linolenic acid;
from 0.35 to 0.65 mg/L, preferably from 0.5 to 0.6 mg/L, more preferably from 0.45 to 0.55 mg/L, of linoleic acid;
from 0.35 to 0.65 mg/L, preferably from 0.5 to 0.6 mg/L, more preferably from 0.45 to 0.55 mg/L, of pipecolic acid;
from 0.5 to 1.5 µg/L, preferably 0.7 to 1.3 µg/L, more preferably 0.9 to 1.1 µg/L, of triiodothyronine;
from 0.35 to 0.65 µg/L, preferably from 0.5 to 0.6 µg/L, more preferably from 0.45 to 0.55 µg/L, of ethanolamine;
from 5 to 12 mg/L, preferably from 6 to 10 mg/L, more preferably from 7 to 9 mg/L, of putrescine;
from 5 to 10 mg/L, preferably from 6 to 9 mg/L, more preferably from 7 to 8 mg/L, of galactose;
from 0.5 to 1.5 mg/L, preferably from 0.7 to 1.3 mg/L, more preferably from 0.8 to 1.1 mg/L, of L-carnitine; and
from 0.8 to 1.8 mg/L, preferably from 1 to 1.5 mg/L, more preferably from 1.2 to 1.3 mg/L, of biotin.

10. A method for the preparation of a nutrient composition, comprising the following steps:
A) providing at least one basal nutrient medium, preferably comprising DMEM/F12, at least one culture medium for cell cultivation in suspension, or a mixture thereof;
B) adjusting therein the amount of at least one antioxidant to a concentration of from 3 to 200 mg/L, preferably from 6 to 64 mg/L, more preferably from 8 to 40 mg/L, more preferably from 8 to 20 mg/L;
C) adjusting therein the amount of sodium selenite to a concentration of from 0.5 to 40 µg/L, preferably from 1 to 25 µg/L, more preferably from 1.5 to 15 µg/L, more preferably from 1.75 to 10 µg/L;
D) adjusting therein the amount of insulin to a concentration of from 1 to 100 mg/L, preferably from 1.8 to 50 mg/L, more preferably from 2 to 20 mg/L, more preferably from 2.4 to 10 mg/L;
E) adjusting therein the amount of transferrin to a concentration of from 0.5 to 20 mg/L, preferably from 1 to 15 mg/L, more preferably from 1.2 to 9 mg/L, more preferably from 1.3 to 5 mg/L; and
F) adjusting therein the amount of at least one fibroblast growth factor receptor agonist, preferably unmodified or modified FGF2 to a concentration of from 4 to 100 µg/L, preferably from 8 to 80 µg/L, more preferably from 10 to 70 µg/L, more preferably from 30 to 40 µg/L;
G) optionally adjusting therein the amount of at least one transforming growth factor beta receptor agonist, preferably modified or unmodified TGFβ1 to a concentration of from 0.1 to 4 µg/L, preferably from 0.2 to 3 µg/L, more preferably from 0.25 to 2 µg/L, more preferably from 0.25 to 1 µg/L; and
H) adjusting therein the amount of albumin, preferably plant albumin or recombinant albumin, to a concentration of from 25 to 1000 mg/L, preferably 50 to 800 mg/L, more preferably 100 to 500 mg/L, more preferably 150 to 350 mg/L; and/or
I) optionally adjusting therein the amount of at least one extract, protein isolate and/or hydrolysate of at least one plant or fungus, preferably yeast extract, to a concentration of from 10 to 5000 mg/L, preferably from 25 to 2000 mg/L, more preferably from 50 to 1500 mg/L, more preferably from 100 to 1000 mg/L.

11. The method according to claim 10, wherein the nutrient composition is the nutrient composition according to any one of claims 1 to 9.

12. A cell culture comprising non-human vertebrate-derived cells and the nutrient composition according to any one of claims 1 to 9 or prepared by the method according to claim 10 or 11, preferably wherein the non-human vertebrate-derived cells are suspended in the nutrient composition.

13. The cell culture according to claim 12, wherein the non-human vertebrate-derived cells are mammalian cells or avian cells, preferably muscle-tissue-derived cells, fat-tissue-derived cells or connective-tissue-derived cells.

14. A method for the cultivation of non-human vertebrate-derived cells, comprising the following steps:
1) introducing vertebrate-derived cells into the nutrient composition according to any one of claims 1 to 9 or prepared by the method according to claim 10 or 11, in order to obtain a cell culture;
2) optionally agitating the cell culture, preferably by stirring, shaking, rocking, and/or inverting;
3) incubating the cell culture;
4) optionally exchanging the nutrient composition in the cell culture with fresh nutrient composition according to any one of claims 1 to 9 or prepared by the method according to claim 10;
5) optionally feeding additional nutrient composition or individual components thereof to the cell culture, depending on consumption thereof.

15. A cellular material obtained by the method according to claim 14.

16. The use of the nutrient composition according to any one of claims 1 to 9 or prepared by the method according to claim 10 or 11, for the cultivation of non-human vertebrate-derived cells, preferably in suspension.

17. A method for the preparation of the nutrient composition according to any one of claims 1 to 8 comprising at least one extract, protein isolate and/or hydrolysate of at least one plant or fungus, preferably yeast extract, wherein the method comprises the steps:
A1) providing the composition according to claim 9; and
B1) mixing the composition provided in step A1) with at least one extract, protein isolate and/or hydrolysate of at least one plant or fungus or with at least one composition comprising a basal nutrient medium and at least one extract, protein isolate and/or hydrolysate of at least one plant or fungus, preferably yeast extract.
